(19) Europäisches Patentamt
European Patent Office
Office européen des brevets

(11) **EP 1 873 667 A2**

(12) **EUROPÄISCHE PATENTANMELDUNG**

(43) Veröffentlichungstag:
**02.01.2008 Patentblatt 2008/01**

(51) Int Cl.:
***G06F 19/00*** *(2006.01)*

(21) Anmeldenummer: **07009101.2**

(22) Anmeldetag: **05.05.2007**

(84) Benannte Vertragsstaaten:
**AT BE BG CH CY CZ DE DK EE ES FI FR GB GR
HU IE IS IT LI LT LU LV MC MT NL PL PT RO SE
SI SK TR**
Benannte Erstreckungsstaaten:
**AL BA HR MK YU**

(30) Priorität: **30.06.2006 DE 102006030210**

(71) Anmelder:
• **Salzsieder, Eckhard
17495 Karlsburg (DE)**

• **Augstein, Petra
17440 Kröslin, OT Freest (DE)**

(72) Erfinder:
• **Salzsieder, Eckhard
17495 Karlsburg (DE)**
• **Augstein, Petra
17440 Kröslin, OT Freest (DE)**

(74) Vertreter: **Meyerhöfer, Dietmar
Patentanwalt,
Rudolf-Petershagen-Allee 12
17489 Greifswald (DE)**

(54) **Verfahren und Anordnung zur rechnergestützten Bestimmung des charakteristischen Tagesprofils des individuellen Glukosemetabolismus**

(57) Die Erfindung betrifft ein Verfahren und eine Anordnung zur rechnergestützten Ermittlung des charakteristischen Tagesprofils der Blutglukosekonzentration in Relation zu individualspezifisch wirksamen Glukose senkenden und Glukose steigernden Einflussgrößen für die Manifestation der aktuellen individuellen Glukosestoffwechselsituation.

Die Aufgabe dieser Erfindung besteht in einem modellbasierten Verfahren sowie einer Anordnung zur Durchführung des Verfahrens, die es rechnergestützt gestattet, für ein jedes Individuum, das heißt sowohl für Probanden mit Prädiabetes oder Metabolischem Syndrom als auch mit Diabetes mellitus Typ 1 oder Diabetes mellitus Typ 2, ein persönliches charakteristisches Tagesprofil des individuellen Glukosemetabolismus in Relation zu individualspezifisch wirksamen, die Blutglukosekonzentration senkenden und die Blutglukosekonzentration steigernden Einflussgrößen zu bestimmen.

Das modellbasierte Verfahren sowie die Anordnung zur Durchführung des Verfahrens zur rechnergestützten Bestimmung des persönlichen charakteristisches Tagesprofil eines jeden Individuums besteht darin, dass für das jeweilige Individuum ein typisches Abbild der aktuellen Glukosestoffwechselsituation als persönlicher Metabolischer Fingerabdruck des individuellen Glukosemetabolismus mit kausal begründeten Beziehungen zwischen dem individuellen Tagesverlauf der Blutglukosekonzentration und den diesen beeinflussenden endogenen und exogenen Stell- und Regelgrößen in qualitativer und quantitativer Form hergestellt wird. Ist das persönliche charakteristisches Tagesprofil der Blutglukosekonzentration eines Individuums einmal ermittelt, können auf der Grundlage des erfindungsgemäß bestimmten charakteristisches Tagesprofil der Blutglukosekonzentration erstmalig kausal begründete Ursachen für das persönlichen charakteristisches Tagesprofil der Blutglukosekonzentration analysiert und individualspezifische Hinweise zur gezielten Beeinflussung des täglichen Verlaufs der Blutglukosekonzentration für dieses Individuum rechnergestützt generiert werden.

Die Erfindung findet in der Gesundheitswirtschaft, bei der Gesundheitsdienstleistung und in der Gesundheitspflege Anwendung.

Fig. 2

EP 1 873 667 A2

## Beschreibung

Die Erfindung betrifft ein Verfahren und eine Anordnung zur rechnergestützten Ermittlung des charakteristischen Tagesprofils der Blutglukosekonzentration in Relation zu individualspezifisch wirksamen Glukose senkenden und Glukose steigernden Einflussgrößen für die Manifestation der aktuellen individuellen Glukosestoffwechselsituation. Die Erfindung findet in der Gesundheitswirtschaft, bei der Gesundheitsdienstleistung und in der Gesundheitspflege Anwendung.

Es ist bekannt, dass die Blutglukosekonzentration eines jeden Individuums über den Tag hinweg in Abhängigkeit von der aufgenommenen Nahrung und den körperlichen Aktivitäten sowie einer Reihe endogener und exogener Stell- und Regelgrößen einen für jedes Individuum bestimmten Verlauf hat. Bei stoffwechselgesunden Individuen wird der Blutglukoseverlauf in sehr engen Grenzen ausgeregelt. Bei verschiedenen Stoffwechselerkrankungen wie z. B. dem Diabetes mellitus unterliegt hingegen der tägliche Blutglukoseverlauf zum Teil erheblichen Schwankungen, die bis hin zu lebensbedrohlichen Situationen führen können.

Bedingt durch die Vielzahl der verschiedenen endogenen und exogenen Einflussgrößen sowie der hohen Komplexität der Interaktionen zwischen den unterschiedlichen Regel- und Stellgrößen ist es schwierig oder gar nicht möglich, die für den jeweiligen Verlauf der Blutglukosekonzentration wesentlichen kausalen Zusammenhänge zu erkennen und gegebenenfalls gezielt zu beeinflussen. Es ist gebräuchlich, die Blutglukosewerte zumeist nur durch punktuelle Messungen zu erfassen, die, wenn überhaupt, in der Regel nur eine sehr grobe Einschätzung des tatsächlichen Blutglukoseverlaufs über den Tag hinweg zulassen.

Das Fehlen geeigneter Identifikations- und Darstellungsformen für die Ermittlung der für jedes Individuum so bedeutungsvollen kausalen Zusammenhänge zwischen dem täglichen Blutglukosekonzentrationsverlauf und den dafür verantwortlichen Glukose senkenden und Glukose steigernden Einflussgrößen für eine Manifestation der aktuellen individuellen Glukosestoffwechselsituation kann somit als eine der wesentlichen Ursachen dafür angesehen werden, dass einerseits frühe Stadien einer sich entwickelnden Stoffwechselerkrankung unerkannt bleiben bzw. nicht rechtzeitig erkannt werden und andererseits die bereits bekannten, hochwirksamen und Kosten sparenden Verfahren, Methoden und Werkzeuge zur Optimierung des individuellen Tagesprofils der Blutglukosekonzentration nicht rechtzeitig oder gar nicht zur Anwendung kommen. Das hat nicht nur gravierende Auswirkungen auf die persönliche Lebenssituation und Lebensqualität der Betroffenen selbst, sondern belastet angesichts der weltweit dramatisch ansteigenden Zahl an Stoffwechselerkrankungen zunehmend das gesamte Gesundheitsversorgungssystem.

Es ist bekannt, dass zur Optimierung der Stoffwechselführung die Kenntnis über die Wechselwirkungen zwischen subkutan verabfolgtem Insulin, aufgenommener Nahrung und den individuellen Blutglukosetagesprofilen unerlässlich ist. Eintritt der Wirkung, Zeitpunkte der Wirkungsmaxima und der Wirkungsdauer in Abhängigkeit von den verabfolgten Insulineinheiten bzw. Broteinheiten der Nahrung sind Faktoren, die die Dynamik und den Verlauf der täglichen Blutglukosekonzentration entscheidend mitbestimmen.

Eine zeit- und situationsgerechte Zuordnung der Einflüsse auf den täglichen individuellen Blutglukosekonzentrationverlauf kann mittels mathematischer Modelle beschrieben werden.

Durch DD-A-230730 und Salzsieder, E. et al.: Computer-aided system in the management of type I diabetes: the application of a model-based strategy, Computer Methods and Programs in Biomedicine, Elsevier Science Publishers Biomedical Division, 1990, Band 32, 215-224 ist ein mathematisches, Modell für den physiologischen Glukose/Insulin-Stoffwechsel in Form eines Differentialgleichungssystems mit den drei Zustandsgrößen Blutglukosekonzentrationsverlauf, endogene Glukosebilanzgröße und Insulinkonzentrationsverlauf bekannt geworden, welches unter Einbeziehung der individualspezifischen Wechselwirkung zwischen Insulin, Nahrung und Blutzuckertagesprofil die Anpassung dieses Modells an die jeweilige Stoffwechselsituation eines an Diabetes mellitus Typ 1 erkranken Probanden mit Zuhilfenahme von routinemäßig erfassten Daten unter klinischen Bedingungen gestattet.

In EP 0 834 825 B1 ist die individualprospektive Bestimmung der Blutglukosekonzentration, der Insulinwirkung und der Nahrungsresorption unter Verwendung eines Simulationsmodells beschrieben. Allein aus den Selbstkontrolldaten des an Diabetes mellitus Typ 1 erkranken Probanden wird mittels eines Mikrorechners das Simulationsmodell an die individualspezifische Wechselwirkung von Insulin, Nahrung und Blutzuckertagesprofil bei diesem Probanden angepasst. Zuerst werden aus den Selbstkontrolldaten mit Hilfe eines Auswertealgorithmus vier probandenspezifische Datensätze selektiert. Der erste Datensatz stellt die im gewählten Beobachtungszeitraum typischer Weise im Tagesverlauf angewandten Insulinapplikationen, der zweite Datensatz die Gesamtmengen der typischer Weise täglich basal und nahrungsbezogen verabfolgten Insuline, der täglich zugeführten Nahrung sowie den BMI, der dritte Datensatz den Zeitpunkt und die Menge der typischer Weise im Tagesverlauf aufgenommene Nahrung und der vierte Datensatz den für den Probanden im betrachteten Zeitraum typischen Tagesverlauf der gemessenen Blutzuckerwerte dar. Aus den im dritten Datensatz enthaltenen Informationen werden mittels eines Identifikationsgleichungssystems, welches eine Beziehung zwischen Selbstkontrolldaten und den Parametern des mathematischen Modells des physiologischen Glukose/Insulin-Stoffwechsels beschreibt, individualspezifische Werte der Modellparameter berechnet. Mit diesen individuellen Modellparameterwerten sowie den Informationen über verabfolgte Insuline und aufgenommener Nahrung erfolgt in einem ersten Iterationsschritt die initiale modellgestützte Berechnung der probandenspezifischen Tagesprofile der Blutglukosekonzentration, der Insulinwirkung und der Nahrungsresorption. Anschließend erfolgt der Vergleich des berechneten Blutglukosetagesprofils mit dem typischen Tagesverlauf der gemessenen Blutglukosewerte. In Abhängigkeit vom Ausgang dieses Vergleichs werden die Wirkprofile des Insulins solange iterativ den Selbstkontrolldaten des Probanden angepasst, bis der Vergleich zwischen den gemessenen Blutglukosewerten und den in zwei aufeinander folgenden Iterationsschritten modellgestützt berechneten Tagesprofilen der Blutglukosekonzentration ausweist, dass gemäß der vorgegebenen Abbruchbedingung keine weitere Verbesserung der Anpassung des Simulationsmodells zur prospektiven Bestimmung der Tagesprofile der Blutglukosekonzentration, der Insulinwirkung und der Nahrungsresorption an die Selbstkontrolldaten des gegebenen Probanden erreicht wird.

Der Nachteil aller bisher bekannten Verfahren zur Bestimmung des charakteristischen Tagesprofils der Blutglukosekonzentration besteht darin, dass deren Anwendung nur auf Diabetes mellitus Typ 1 erkrankte Probanden beschränkt ist.

Die Aufgabe dieser Erfindung ist es daher, ein modellbasiertes Verfahren sowie eine Anordnung zur Durchführung des Verfahrens darzustellen, die es rechnergestützt gestattet, das für ein jedes Individuum, das heißt sowohl bei Probanden mit Prädiabetes oder Metabolischem Syndrom als auch mit Diabetes mellitus Typ 1 oder Diabetes mellitus Typ 2, sein persönliches charakteristisches Tagesprofil des individuellen Glukosemetabolismus in Relation zu individualspezifisch wirksamen, die Blutglukosekonzentration senkenden und die Blutglukosekonzentration steigernden Einflussgrößen zu bestimmen.

Erfindungsgemäß wird die Aufgabe durch ein modellbasiertes Verfahren sowie eine Anordnung zur Durchführung des Verfahrens zur rechnergestützten Bestimmung des persönlichen charakteristisches Tagesprofil eines jeden Individuums gelöst, welches für das jeweilige Individuum ein typisches Abbild der aktuellen Glukosestoffwechselsituation als persönlicher Metabolischer Fingerabdruck des individuellen Glukosemetabolismus mit kausal begründeten Beziehungen zwischen dem individuellen Tagesverlauf der Blutglukosekonzentration und den diesen beeinflussenden endogenen und exogenen Stell- und Regelgrößen in qualitativer und quantitativer Form herstellt. Ist das persönliche charakteristisches Tagesprofil der Blutglukosekonzentration eines Individuums einmal ermittelt, können auf der Grundlage des erfindungsgemäß bestimmten charakteristisches Tagesprofil der Blutglukosekonzentration in Relation zu individualspezifisch wirksamen Glukose senkenden und Glukose steigernden Einflussgrößen erstmalig kausal begründete Ursachen für das persönlichen charakteristisches Tagesprofil der Blutglukosekonzentration analysiert und individualspezifische Hinweise zur gezielten Beeinflussung des täglichen Verlaufs der Blutglukosekonzentration für dieses Individuum rechnergestützt generiert werden.

Das erfindungsgemäße Verfahren zur rechnergestützten Bestimmung des persönlichen charakteristisches Tagesprofil der Blutglukosekonzentration eines Individuums in Relation zu den individualspezifisch wirksamen Glukose senkenden und Glukose steigernden Einflussgrößen besteht darin, dass in einem ersten Verfahrensschritt mittels eines mathematischen Modells zur Beschreibung des physiologischen Glukosemetabolismus mit den vier Zustandsgrößen Blutglukosekonzentrationsverlauf

$$dx/dt = u(t) + (BE(t) - OAD_1(t)) \tag{1}$$

endogene Glukosebilanz

$$du/dt = - (b_1+b_2)u(t) - b_3(y(t) + e(t) + OAD_2(t)) + b_1(b_0 - b_{0k}(BE(t)) - BE(t) + OAD_3(t)) \tag{2}$$

endogener Insulinmetabolismus

$$dy/dt = - k(y(t)) + [a_0 + a_1(x(t) - x_w) + a_2(BE(t)/dt)] + OAD_4(t) + IE_{exog}(t) \tag{3}$$

und
Exercise induzierte Glukoseverwertung

$$de/dt = - k(e(t)) + IDE(t) \tag{4}$$

aus vorhandenen Angaben eines Individuums zu seinen typischerweise täglich aufgenommenen Broteinheiten (BE), seinen Medikationen mit oral verabfolgten Antidiabetika unterschiedlicher Wirkklassen ($OAD_i$) und Insulin (IE) sowie seinen sportlichen Aktivitäten (IDE) der persönliche tägliche Blutglukosekonzentrationsverlauf x(t) dieses Individuums in Relation zu den endogenen Bilanzgrößen Glukosebilanz u(t) und Insulinmetabolismus y(t) sowie der Exercise induzierten Glukoseverwertung e(t) mittels eines Mikrorechnersystems berechnet wird, wobei für die Berechnung des persönlichen charakteristischen Tagesprofils der Blutglukosekonzentration die individualspezifischen Parameterwerte $b_i$, $a_i$ und k des Modellgleichungssystems Anwendung finden. Eine gewisse Besonderheit besteht hierbei hinsichtlich der Insulinzufuhr IE, die sich aus einem exogenen, d.h. von außen zuzuführenden körperfremden Anteil $IE_{exog}$ und einem körpereigenen, d.h. von innen zuzuführenden endogenen Anteil

$$IE_{endog} = [a_0 + a_1(x(t) - x_w) + a_2(BE(t)/dt)] \qquad (3.1)$$

additiv zu der Gesamtinsulinzufuhr

$$IE(t) = [IE_{exog}(t) + IE_{endog}(t)]\, F_n(t) \qquad (3.2)$$

zusammensetzt und mit den individualspezifischen Faktoren $F_n(t)$ zur Berücksichtigung des individuellen Tagesgangs der Insulinwirkung multipliziert wird.

Die Bestimmung der individualspezifischen Parameterwerte $b_i$, $a_i$ und k des erfindungemäßen Modellgleichungssystems mit den Gleichungen (1) bis (4) und den Faktoren $F_n$ eines Individuums erfolgt vorzugsweise iterativ mittels bekannter mathematischer Optimierungsverfahren. Den physiologisch interpretierbaren Parametern des Modellgleichungssystems sind kausal die nachstehenden metabolischen Vorgänge

$b_0$ = endogener Glukosezustrom
$b_{0k}$ = Wirkfaktor für die BE-abhängige Verminderung des endogenen Glukosezustroms
$b_1$ = Verstärkungsfaktor für den endogenen Glukoseumsatz
$b_2$ = Zeitkonstante für den endogenen Glukoseumsatz
$b_3$ = Wirkfaktor für die insulingetriggerte Glukoseverwertung
$a_0$ = glukoseunabhängige endogene Insulinbereitstellung
$a_1$ = Wirkfaktor für die glukosegesteuerte endogene Insulinbereitstellung
$a_2$ = Wirkfaktor für die BE-gesteuerte endogene Insulinbereitstellung
$x_w$ = Glukosekonzentration, bei der die endogene Insulinbereitstellung zu Null wird
k = Wirkfaktor für den Insulinkatabolismus

zugeordnet.

Die individualspezifische Berechnung des täglichen Blutglukosekonzentrationsverlaufs x(t) eines Individuums erfolgt mittels dieses Modellgleichungssystems vorzugsweise im 1- Minutentakt, sodass insgesamt 1440 Einzelwerte zu bestimmen sind, die nach ihrer wertmäßigen Ermittlung sequenziell auf einer 24-Stunden-Tageszeitskala von 0:00 Uhr bis 24:00 Uhr abgebildet werden.

In einem zweiten Verfahrensschritt werden erfindungsgemäß die persönlichen 24-Stunden-Wirk- bzw. 24-Stunden-Anflutungsprofile IE(t), BE(t), OAD(t) und IDE(t) für die Eingangsgrößen Insulinzufuhr (IE), Kalorienzufuhr (BE), Applikation oraler Antidiabetika (OAD) und körperliche Aktivität (IDE) für ein Individuum bestimmt.

Zur rechnergestützten Bestimmung des persönlichen 24-Stunden-Insulinwirkprofils IE(t) wird zunächst quasi kontinuierlich im Minutentakt der körpereigene Anteil $IE_{endog}(t)$ gemäß Gleichung 3.1 berechnet und auf der 24-Stunden-Tageszeitskala von 0:00 Uhr bis 24:00 Uhr abgebildet.

Die Berechnung des 24-Stunden-Insulinwirkprofils des exogenen Anteils $IE_{exog}(t)$ erfolgt in fünf Bearbeitungsschritten.

In einem ersten Bearbeitungsschritt werden die individuellen Angaben zur Applikation körperfremden Insulins nach Tageszeit, Insulinart und Dosis des betreffenden Individuums in eine im Rechner angelegte Selbstkontrolldatentabelle eingetragen und gespeichert.

In einem zweiten Bearbeitungsschritt werden die Angaben zur Insulinart mit einer im Rechner gespeicherten Insulintabelle verglichen. In dieser Insulintabelle sind alle handelsüblichen Insulinpräparate nach 5 Wirkklassen geordnet abgelegt. Diese Wirkklassen sind kurzwirksame Insuline, Normalinsuline, Semilenteinsuline, Basalinsuline und Mischinsuline. Mit Ausnahme der Mischinsuline ist jeder Wirkklasse ein normiertes Grundwirkprofil für 1 IE mit den Kernangaben zu Wirkungsstärke und Wirkungsdauer zugeordnet, welche gleichfalls in der Insulintabelle abgelegt sind. Bei Übereinstimmung zwischen einer in der Selbstkontrolldatendatei abgelegten Insulinart und einem Insulinpräparat aus einer der 5 Wirkklassen in der Insulintabelle, werden die entsprechenden Angaben für dieses Insulinpräparat aus der Insulintabelle selektiert und im Rechner zwischengespeichert.

Sind alle exogen verabfolgten Insulinpräparate identifiziert, erfolgt in einem dritten Bearbeitungsschritt die multiplikative Hochrechnung des Grundwirkungsprofils von 1 IE auf die tatsächlich verabfolgte Dosis gemäß den Angaben aus der Selbstkontrolldatentabelle. Diese Hochrechnung erfolgt flächenäquidistant, wobei der jeweilige Dosismultiplikator zu jeweils 50% auf die Wirkungsstärke und auf die Wirkungsdauer verteilt wird.

Handelt es sich bei dem ausgewählten Insulinpräparat um ein Mischinsulin, muss vor der Hochrechnung noch die Zusammensetzung des Mischinsulins bestimmt werden. Hierzu wird zunächst aus einer gesonderten Tabelle, in welcher die Zusammensetzungen handelsüblicher Mischinsuline hinsichtlich der Anteile an kurz- und langwirksamen Insulinen in den Relationen 90:10, 80:20, 70:30, 60:40 und 50:50 abgelegt sind, die zutreffende Zusammensetzung ausgewählt. Anschließend erfolgt die additive Zusammenführung aus den normierten Grundprofilen der Einzelbestandteile des Mischinsulins gemäß der ausgewählten Relation zu einem entsprechenden Mischinsulinwirkprofil. Die dosisbezogene Hochrechnung erfolgt dann analog zu den ungemischten Insulinpräparaten.

In einem vierten Bearbeitungsschritt werden die so berechneten und zwischengespeicherten Einzelinsulinwirkprofile entsprechend den aus der Selbstkontrolldatentabelle zu entnehmenden Verabfolgungszeiten zu einem 24-Stunden-Insulinwirkprofil $IE_{exog}(t)$ für den exogenen Anteil am gesamten 24-Stunden-Insulinwirkprofil $IE(t)$ des persönlichen charakteristischen Tagesprofil des individuellen Glucosemetabolismus eines Individuums zusammengeführt.

In einem abschließenden fünften Bearbeitungsschritt wird auf das gemäß Gleichung 3.2 additiv zu einem Gesamtinsulinwirkprofil $IE(t) = IE_{endog}(t) + IE_{exog}(t)$ zusammengeführte 24-Stunden-Insulinwirkprofil $IE(t)$ der zuvor für jedes Individuum ermittelte, individualspezifische Tagesgang in der Insulinwirkung aufmultipliziert. Dieser individualspezifische Tagesgang der Insulinwirkung ist in Form von 24 Multiplikationsfaktoren $F_1$ bis $F_{24}$ ($F_n$), die die Veränderungen in der Insulinwirkung pro Stunde über den Tag hinweg von 0:00 Uhr bis 24:00 Uhr beschreiben, für jedes Individuum im Rechner in einem gesonderten Sektor der Selbstkontrolldatentabelle abgespeichert. Der Wertebereich der dimensionslosen Multiplikationsfaktoren $F_n$ bewegt sich zwischen 0,5 und 2,0, wobei ein Wert von 1,0 anzeigt, dass für den entsprechenden Zeitabschnitt keine Veränderungen der Insulinwirkung gegenüber den ursprünglich berechneten 24-Stunden-Insulinwirkprofilen $IE(t)$ vorliegt.

Die Berechnung des 24-Stunden-BE-Anflutungsprofils $BE(t)$ eines Individuums erfolgt in drei Bearbeitungsschritten.

In einem ersten Bearbeitungsschritt werden die individuellen Angaben zur BE-Aufnahme hinsichtlich der Menge der zu den jeweiligen Mahlzeiten aufgenommenen BE und zu den Zeitpunkten der BE-Aufnahmen in die Selbstkontrolldatentabelle eingetragen und dort gespeichert. Zur Bestimmung der für eine Mahlzeit zutreffenden BE ist zudem im Rechner eine BE-Tabelle angelegt, die eine schnelle und unkomplizierte Zuordnung der BE zu einer Vielzahl von Nahrungsmitteln erlaubt und zudem eine Umrechnung der BE in Kohlenhydrateinheiten mit Angaben zum Energiegehalt ermöglicht.

In einem zweiten Bearbeitungsschritt wird das der jeweiligen Nahrungsaufnahe zugeordnete BE-Anflutungsprofil in Relation zu einem in der BE-Tabelle abgespeicherten normierten Anflutungsgrundprofil für 1 BE mit Angaben zur Anflutungshöhe und zur Anflutungsdauer berechnet. Die Hochrechnung von dem 1-BE-Anflutungsgrundprofil auf das Profil, welches den tatsächlich aufgenommenen BE entspricht, erfolgt analog zu den Insulinwirkprofilen flächenäquidistant.

In einem dritten Bearbeitungsschritt werden die so berechneten einzelnen BE-Anflutungsprofile schließlich zu dem 24-Stunden-BE-Anflutungsprofil $BE(t)$ im persönlichen charakteristischen Tagesprofil des individuellen Glucosemetabolismus eines Individuums zusammengeführt und entsprechend den zeitlichen Angaben aus der Selbstkontrolldatentabelle auf die 24-Stunden-Tagesskala zwischen 0:00 Uhr und 24:00 Uhr abgebildet.

Die Berechnung des persönlichen 24-Stunden-Wirkprofils oral aufgenommener Antidiabetika $OAD(t)$ eines Individuums erfolgt erfindungsgemäß in drei Bearbeitungsschritten.

In einem ersten Bearbeitungsschritt werden die individuellen Angaben zur Applikation oral verabfolgter Antidiabetika nach Tageszeit, OAD-Formulation und verabreichter Dosis des betreffenden Individuums in der Selbstkontrolldatentabelle erfasst und gespeichert.

In einem zweiten Bearbeitungsschritt werden die Angaben zur OAD-Formulation und zur verabreichten Dosis mit einer im Rechner gespeicherten OAD-Tabelle verglichen. In dieser OAD-Tabelle sind alle handelsüblichen oralen Antidiabetika differenziert nach fünf Wirkklassen abgelegt. Diese fünf Wirkklassen sind Alphaglukosidasehemmer, Insulinsensitizer, Biguanide, Sulphonylharnstoffe und Glinide. Jedem dieser fünf Wirkklassen sind auf die jeweilige Basisdosis normierte Grundwirkprofile mit Angaben zur Wirkstärke und zur Wirkdauer zugeordnet. Wird die Dosis des jeweiligen oralen Antidiabetikums erhöht, wirkt sich diese Erhöhung proportional auf die Wirkstärke aus. Die Wirkdauer bleibt unbeeinflusst. Bei Übereinstimmung zwischen einem der angegebenen OAD-Formulationen mit einem OAD-Präparat aus der OAD-Tabelle wird das entsprechende, dosisbezogene Wirkprofil dieses OAD-Präparats aus der Tabelle selektiert und für den nachfolgenden Bearbeitungsschritt bereitgestellt.

In einem dritten Bearbeitungsschritt werden die einzelnen OAD-Wirkprofile entsprechend den zeitlichen Angaben aus der Selbstkontrolldatentabelle auf die 24-Stunden-Tageszeitskale abgebildet, wodurch über den Tag hinweg schließlich das OAD-Gesamtwirkprofil OAD(t) des persönlichen charakteristischen Tagesprofils des individuellen Glukosemetabolismus entsteht.

Die Berechnung des individuellen 24-Stunden-IDE-Wirkprofils IDE(t) erfolgt erfindungsgemäß in Form von individualspezifischen Insulinwirkäquivalenten (IDE = Insulin Dose Equivalent) körperlicher Aktivitäten in drei Bearbeitungsschritten.

Hierzu werden in einem ersten Bearbeitungsschritt die individuellen Angaben zu körperlichen Aktivitäten in der Selbstkontrolldatentabelle nach Zeitpunkt, Art, Intensität und Dauer erfasst und gespeichert.

Um aus diesen Angaben individualspezifische Insulinwirkäquivalente IDE in Form von Insulineinheiten (IE) und deren Wirkprofile IDE(t) berechnen zu können, werden diese Angaben in einem zweiten Bearbeitungsschritt auf ein IDE-Standardprofil mit der Wirkung von einer IE normiert. Hierzu stehen in einer IDE-Tabelle drei Arten körperlicher Aktivitäten Laufen, Schwimmen und Radfahren und die drei Intensitätsgrade leicht, mittel und schwer zur Auswahl zur Verfügung. Jeder Art körperlicher Aktivität und jedem Intensitätsgrad sind Multiplikationsfaktoren zugeordnet, welche zusammengenommen die Wirkstärke des gesuchten IDE-Profils bestimmen. Das Integral der zwischen Wirkstärke und gewählter Wirkdauer aufgespannten Wirkfläche entspricht dem für die ausgewählte körperliche Aktivität gesuchten IDE-Wirkprofil IDE(t).

In einem dritten Bearbeitungsschritt werden schließlich die so berechneten IDE-Einzelwirkprofile entsprechend den aus der Selbstkontrolldatentabelle zu entnehmenden Zeitpunkten körperlicher Aktivitäten entlang der Tageszeitskala zu dem 24-Stunden-IDE-Wirkprofil IDE(t) des persönlichen charakteristischen Tagesprofil des individuellen Glukosemetabolismus eines Individuums zusammengeführt.

In einem dritten Verfahrenschritt wird ein individualspezifisches Referenzblutglukosetagesprofil BG(t) aus gemessenen Blutkosewerten zur Validitätsprüfung des modellgestützt berechneten persönlichen Blutglukosekonzentrationsverlaufs x(t) eines Individuums generiert und der Startwert x(0)t für das numerische Integrationsverfahren erzeugt. Die Generierung des Referenzblutglukosetagesprofils BG(t) wird erfindungsgemäß dadurch erreicht, dass die Blutglukosereferenzwerte $BG_n$ eines Individuums vorzugsweise quasikontinuierlich in einem 5 Minutenzeitraster über einen Zeitraum von bis zu 72 Stunden gemessen und aufgezeichnet werden. Aus den bis zu 864 aufgezeichneten Einzelmesswerten wird anschließend unter Anwendung bekannter Verfahren der mathematischen Statistik und Mittelwertsbildung das Referenzblutglukosetagesprofil BG(t) mit 288 Messpunkten erzeugt und in äquidistanten Zeitabständen von 5 Minuten auf der 24-Stunden-Tageszeitskala von 0:00 Uhr bis 24:00 Uhr abgebildet. Die Generierung des Starwertes x(0)t erfolgt durch Berechnung des Tagesmittelwertes des Refenzblutglukosetagesprofils gemäß der bekannten mathematischen Beziehung $BG_{mittel}$ = Summe $BG_n$/n, wobei der berechnete Mittelwert der Tagesblutglukosekonzentration gleich dem Startwert x(0)t für das numerische Integrationsverfahren zur modellgestützte Berechnung des individualspezifischen Blutglukosekonzentrationsverlaufs x(t) eines Individuums gesetzt wird.

In einem vierter Verfahrensschritt werden die 24-Stunden-Profile des modellgestützt berechneten Blutglukosekonzentrationsverlaufs x(t), der Insulinwirkung IE(t), der BE-Anflutung BE(t), der Wirkung oraler Antidiabetika OAD(t), der körperlichen Aktivitäten IDE(t) und des gemessenen Referenzblutglukosetagesprofils BG(t) eines Individuums über der Tageszeitskala von 0:00 Uhr bis 24:00 Uhr unter Verwendung der entsprechenden Angaben aus der Selbstkontrolldatentabelle synchronisiert und erfindungsgemäß zu dem persönlichen charakteristischen Tagesprofil des individuellen Glukosemetabolismus dieses Individuums im Sinne des Metabolischen Fingerabdrucks seines Glukosmetabolismus zusammengeführt und in einer Ausgabedatei zur weiteren Verwendung gespeichert.

Zudem werden die insgesamt pro Tag verabreichten Insulineinheiten IE/Tag mit differenziertem Ausweis für den endogenen und den exogen Anteil durch Integration der jeweiligen Insulinwirkflächen, die pro Tag aufgenommenen BE/Tag durch Integration der BE-Anflutungsfläche, der Anzahl von OAD-Tabletten/Tag durch Integration der OAD-Wirkflächen sowie die Gesamtwirkung körperlicher Aktivitäten IDE/Tag durch Integration der IDE-Wirkfläche ermittelt und gleichfalls in der Ausgabedatei gespeichert.

In einem fünften Verfahrensschritt wird erfindungsgemäß das persönliche charakteristische Tagesprofil des individuellen Glukosemetabolismus eines Individuums mittels bekannter Methoden der grafischen Präsentation visualisiert. Um das zu erreichen, werden die sechs 24-Stunden-Profile x(t), IE(t), BE(t), OAD(t), IDE(t) und BG(t) des in der Ausgabedatei gespeicherten persönlichen charakteristischen Tagesprofil des individuellen Glukosemetabolismus eines Individuums über einer einheitlichen Tageszeitskala von 0:00 Uhr bis 24:00 Uhr gemäß ihrer jeweiligen spezifischen quantitativen und qualitativen Eigenschaften grafisch dargestellt. Die Darstellung erfolgt von oben nach unten betrachtet in der Sequenz Blutglukosekonzentrationen, Insulinwirkprofile, BE-Anflutungsprofile, OAD-Wirkprofile, IDE-Wirkprofile und Tageszeitskala.

Der modellgestützt berechnete 24-Stunden-Blutglukosekonzentrationsverlauf x(t) und das gemessene Referenzblutglukosetagesprofil BG(t) des persönlichen charakteristischen Tagesprofils des individuellen Glukosemetabolismus eines Individuums werden zusammen in dem dafür vorgesehenen erfindungsgemäßen Grafikteil als Konzentrationsverläufe wahlweise in der Dimension mmol/l oder mg/dl in der Form einer ausgezogene Linie (x(t)) sowie einer gepunkteten Linie (BG(t)) dargestellt. Zudem wird in diesem Grafikteil ein Blutglukosekonzentrationsreferenzbereich eingeblendet, welcher den normalerweise üblichen Konzentrationsbereich der Blutglukose bei stoffwechselgesunden Individuen über den Tag hinweg aufzeigt. Die prozentualen, auf den Tag bezogenen Anteile der modellgestützt berechneten Blutglukosekonzentrationswerte x(t), die den Referenzbereich unter- oder überschreiten werden mittels einer Tortengrafik im Sinne eines Qualitätsauges in der rechten oberen Ecke des persönlichen charakteristischen Tagesprofils des individuellen Glukosemetabolismus eines Individuums mit prozentualer Angabe zu den Glukosewerten innerhalb und außerhalb des Referenzbereiches gesondert dargestellt.

Die Insulinwirkprofile IE(t) des persönlichen charakteristischen Tagesprofils des individuellen Glukosemetabolismus eines Individuums werden additiv für die beiden Wirkprofile $IE_{endog}(t)$ und $IE_{exog}(t)$ in der Dimension Insulineinheiten grafisch dargestellt, wobei zur besseren Differenzierung für die Darstellung der beiden Insulinwirkprofilanteile $IE_{endog}(t)$ und $IE_{exog}(t)$ differenziert ausgewiesen werden. Zu dem Wirkprofil $IE_{exog}(t)$ werden zu den Applikationszeiten schmale Rechtecksäulen eingeblendet, deren Flächengestaltung mit der Insulinwirkklasse und deren Höhe mit der Anzahl der verabreichten Insulineinheiten korrespondieren. Diesem Grafikteil zugeordnet werden auch die gesamten pro Tag verabfolgten IE, differenziert nach exogenem und endogenem Insulin, in numerischer Form aufgeführt.

Die grafische Darstellung der BE-Anflutungsprofile BE(t) erfolgt in der Dimension Broteinheiten (BE). An den Zeitpunkten im Tagesablauf, an welchen gegessen wird, d.h. zu den Zeitpunkten, bei denen die jeweilige BE-Anflutung beginnt, werden schmale Rechtecksäulen eingeblendet, deren Höhe der Anzahl der aufgenommenen BE entspricht. Zudem werden diesem Grafikteil zugeordnet die insgesamt pro Tag aufgenommenen BE in numerischer Form eingeblendet.

Das OAD-Anflutungsprofil OAD(t) des persönlichen charakteristischen Tagesprofils des individuellen Glukosemetabolismus eines Individuums wird analog dem BE-Anflutungsprofil BE(t) dargestellt, wobei aber die Dimension der grafischen Darstellung des OAD-Anflutungsprofils OAD(t) der Anzahl der eingenommenen Tabletten entspricht. Anstelle der Rechtecksäulen in der grafischen Darstellung des BE-Anflutungsprofils BE(t) sind in der grafischen Darstellung des OAD-Anflutungsprofils OAD(t) zu den Zeiten der Tabletteneinnahme symbolhaft kleine Kreise angeordnet, deren Anzahl der Anzahl der eingenommenen Tabletten entspricht. Zur Differenzierung der unterschiedlichen OAD-Wirkklassen wird jeder Wirkklasse eine wirkklassentypische Flächenkennzeichnung der Kreise zugeordnet.

Für die grafische Darstellung des Wirkprofils körperlicher Aktivitäten in der Dimension Insulineinheitenäquivalente (IDE) im persönlichen charakteristischen Tagesprofil des individuellen Glukosemetabolismus eines Individuums wird zu dem eigentlichen IDE-Wirkprofil IDE(t) zu den Zeitpunkten der körperlichen Aktivitäten zusätzlich ein Rechteck eingeblendet, dessen Höhe der Intensität und dessen Breite der Dauer der jeweiligen körperlichen Aktivität entspricht. Zudem werden die insgesamt über den Tag hinweg wirksamen Insulinwirkäquivalente diesem Grafikteil in numerischer Form zugeordnet dargestellt.

Zur Visualisierung und Kenntlichmachung, welche ursächlichen Zusammenhänge zwischen den berechneten und dargestellten 24-Stunden-Wirk- und 24-Stunden-Anflutungsprofilen IE(t), BE(t), OAD(t) und IDE(t) und dem 24-Stunden-Blutglukosekonzentrationsverauf x(t) des persönlichen charakteristischen Tagesprofils des individuellen Glukosemetabolismus eines Individuums bestehen, werden zwischen den Wirk- und Anflutungsprofilen und den entsprechend markierten Bereichen im Blutglukosekonzentrationsverlauf, für die ein derartiger kausaler Zusammenhang besteht, Beziehungspfeile in die grafische Darstellung des persönlichen charakteristischen Tagesprofil des individuellen Glukosemetabolismus eingeblendet.

Zur rechnergestützten Bestimmung des charakteristischen Tagesprofils des individuellen Glukosemetabolismus (CTP) eines Individuums in der Qualität eines Metabolischen Fingerabdrucks seines individuellen Glukosemetabolismus kommt eine Mikrorechneranordnung zur Anwendung, die erfindungsgemäß aus einem Mikrorechnerssystem mit einem CTP-Eingangsgrößenmodul, einem nachgeordneten CTP-Berechnungsmodul, an das ein CTP-Darstellungsmodul angeschlossen ist, besteht.

Das CTP-Eingangsgrößenmodul stellt die Schnittstelle zur Eingabe der individualspezifischen Parameterwerte $b_i$, a; und k für das Modellgleichungssystem, der Selbstkontrolldaten IE, BE, OAD, IDE, der Multiplikationsfaktoren $F_n$ für den Tagesgang der Insulinwirkung sowie der gemessenen Blutglukosewerte BG eines Individuums dar. Mittels dieses CTP-Eingangsgrößenmoduls werden die individualspezifischen Parameterstartwerte $b_i$, $a_i$ und k des mathematischen Simulationsmodells, die individuellen Selbstkontrolldaten IE, BE, OAD und IDE, die individuellen Multiplikationsfaktoren $F_n$ für den Tagesgang der Insulinwirkung und die gemessenen Blutglukosewerte BG eines Individuums zugeführt und als Eingangsgrößen anschließend an das nachgeordnete CTP-Berechnungsmodul übergeben, in welchem die 24-Stunden-Profile der Blutglukosekonzentration x(t), der Insulinwirkung IE(t), der BE-Anflutung BE(t), der Wirkung oraler Antidiabetika OAD(t), der insulinäquivalenten Wirkung körperlicher Aktivitäten IDE(t) und das Referenzblutglukosetagesprofil BG(t) des persönlichen charakteristischen Tagesprofils des individuellen Glukosemetabolismus eines Individuums berechnet werden. Die Ergebnisse dieser Berechnungen werden danach einem CTP-Darstellungsmodul zugeführt, mit dessen Hilfe die Ergebnisse der Berechnungen mit der Qualität eines Metabolischen Fingerabdrucks visualisiert werden.

Das CTP-Eingangsgrößenmodul besteht aus sechs Komponenten, einer Modellparameterkomponente, einer Insulinapplikationskomponente, einer Nahrungsaufnahmekomponente, einer Komponente für Applikation oraler Antidiabetika, einer Komponente für die körperlichen Aktivitäten und einer Referenzblutglukosemesswertekomponente. Jede dieser sechs Komponenten des CPT-Eingangsmoduls ist eingangsseitig mit einer ihr zugeordneten Schnittstelle verbunden, an der jeweils eine der sechs Eingabegrößen Parameterwerte $b_i$, $a_i$ und k, Selbstkontrolldaten IE, BE, OAD und IDE, Multiplikationsfaktoren $F_n$ und Blutglukosemesswerte BG angelegt ist. Jede Eingabegröße wird über einen Eingang des CTP-Eingangsgrößenmoduls dem für sie zugeordneten Sektor der Selbstkontrolldatentabelle zugeführt und dort in der Selbstkontrolldatentabelle des CTP-Eingangsgrößenmoduls abgespeichert.

Über die erste Eingabeschnittstelle wird die Eingangsgröße Parameterwerte $b_i$, $a_i$, und k in Form dimensionsloser numerischer Werte dem Eingang der Modellparameterkomponente des CTP-Eingangsgrößenmoduls geführt und dort in dem der Modellparameterkomponente zugeordneten Sektor der Selbstkontrolldatentabelle des CTP-Eingangsgrößenmoduls gespeichert.

Die Eingabe der Eingangsgröße Selbstkontrolldaten Insulinapplikationen (IE) erfolgt über die zweite Eingabeschnittstelle, die mit dem Eingang der Insulinapplikationskomponente des CTP-Eingangsgrößenmoduls verbunden ist. Die Eingabe der Eingangsgröße IE erfolgt in Tripelform, wobei die ersten Tripel der Insulindosis, die zweiten Tripel der Insulinart und die dritten Tripel den Applikationszeiten zugeordnet sind. Die Speicherung der IE in dem der Insulinapplikationskomponente entsprechenden Sektor der Selbstkontrolldatentabelle des CTP-Eingangsgrößenmoduls erfolgt gleichfalls in Tripelform. Zudem werden über diese zweite Eingabeschnittstelle die Multiplikationsfaktoren $F_n$ für den Tagesgang der Insulinwirkung an den Eingang der Insulinapplikationskomponente des CTP-Eingangsgrößenmoduls gelegt und in einem gesonderten Sektor der Selbstkontrolldatentabelle gespeichert.

Die Eingangsgröße Selbstkontrolldaten Nahrungsaufnahme (BE) wird über eine dritte Eingabeschnittstelle in Dualform an den Eingang der Nahrungsaufnahmekomponente des CTP-Eingangsgrößenmoduls geführt und in dem dieser Komponente entsprechenden Sektor der Selbstkontrolldatentabelle des CTP-Eingangsgrößenmoduls gespeichert. Die Dualform umfasst als Dualteil 1 die Menge der BE und als Dualteil 2 die Tageszeit, zu der die BE aufgenommen werden.

Über eine vierte Eingabeschnittstelle wird die Eingangsgröße Selbstkontrolldaten oral verabfolgte Antidiabetika (OAD) an den Eingang der Komponente Applikation oraler Antidiabetika des CTP-Eingangsgrößenmoduls gelegt und in dem dieser Komponente entsprechenden Sektor der Selbstkontrolldatentabelle abgespeichert. Die Eingabe und Abspeicherung der Eingangsgröße OAD erfolgt in Tripelform, wobei die ersten Tripel den AOD-Formulationen, die zweiten Tripel den Dosisangaben und die dritten Tripel den Applikationszeiten zugeordnet sind.

Die Eingabe der Eingangsgröße Selbstkontrolldaten Insulinwirkäquivalente körperlicher Aktivitäten (IDE) erfolgt über eine fünfte Eingabeschnittstelle. Die in Tripelform einzugebenden IDE, wobei die ersten Tripel den Intensitäten, die zweiten Tripel den Dauern und die dritten Tripel den Zeitpunkten des Beginns der jeweiligen körperlichen Aktivitäten zugeordnet sind, werden an den Eingang der Komponente für körperliche Aktivitäten geführt und in dem dieser Komponente entsprechenden Sektor der Selbstkontrolldatentabelle des CTP-Eingangsgrößenmoduls abgelegt.

Über eine sechste Eingabeschnittstelle werden die gemessenen Blutglukosewerte (BG) in der Dimension mmol/l mit Angabe der Messzeit, d.h. in Dualform an den Eingang der Referenzblutglukosemesswertekomponente des CTP-Eingangsgrößenmoduls geführt und in dem der Komponente entsprechenden Sektor den Selbstkontrolldatentabelle des CTP-Eingangsgrößenmoduls gespeichert.

Dem CTP-Eingangsgrößenmodul sind in der erfindungsgemäßen Anordnung in linearer Folge sechs Berechnungseinheiten des CTP-Berechnungsmoduls nachgeschaltet. Dabei ist jeweils ein Ausgang der sechs Ausgänge des CTP-Eingangsgrößenmoduls mit einem Eingang der ersten sechs Berechnungseinheiten des CTP-Berechnungsmoduls verbunden. Die erste Berechnungseinheit ist die 24-Stunden-Modellgleichungs-Berechnungseinheit, die zweite Berechnungseinheit ist die 24-Stunden-Insulinwirkprofil-Berechnungseinheit (IE(t)), die dritte Berechnungseinheit ist die 24-Stunden-BE-Anflutungs-Berechnungseinheit (BE(t)), die vierte Berechnungseinheit ist die 24-Stunden-OAD-Wirkprofil-Berechnungseinheit (OAD(t)), die fünfte Berechnungseinheit ist die 24-Stunden-IDE-Wirkprofil-Berechnungseinheit (IDE(t)) und die sechste Berechnungseinheit ist die Referenzblutglukosetagesprofil-Berechnungseinheit (BG(t)). Der Ausgang dieser sechs Berechnungseinheiten ist jeweils mit einem der sechs Eingänge der nachgeordneten CTP-Summations-Berechnungseinheit (x(t)) als siebte Berechnungseinheit des CTP-Berechnungs-Moduls verknüpft.

In der Modellgleichungs-Berechnungseinheit ist das erfindungsgemäße mathematische Modell gemäß den Modellgleichungen (1) bis (4) mit seinen vier Zustandsgrößen Blutglukosekonzentrationsverlauf (dx/dt), endogene Glukosebilanz (du/dt), endogener Insulinkatabolismus (dy/dt) und Exercise induzierte Glukoseverwertung (de/dt) zur Berechnung des 24-Stunden-Blutglukosekonzentrationsverlaufs x(t) eines Individuums angeordnet. Die Berechnung des 24-Stunden-Blutglukosekonzentrationsverlaufs x(t) eines Individuums erfolgt durch numerische Integration des Modellgleichungssystems, wobei für den Integrationsalgorithmus vorzugsweise eine Schrittweite von 1 Minute gewählt wird, so dass über den Tag hinweg 1440 Einzelwerte zu bestimmen sind. Um den individuellen 24-Stunden-Blutglukosekonzentrationsverlauf x(t) eines Individuums berechnen zu können, müssen die individualspezifischen Modellparameterwerte $b_i$, $a_i$ und k des jeweiligen Individuums in das Modellgleichungssystem eingefügt werden. Diese individuellen Modellparameterwerte werden der Modellgleichungs-Berechnungseinheit über den ersten Eingang des CTP-Berechnungsmoduls zugeführt, welcher mit dem ersten Ausgang der Modellparameterkomponente des CTP-Eingangsgrößenmoduls verbunden ist, in welcher die individuellen Modellparameterwerte in dem der Modellparameterkomponente zugeordneten Sektor der Selbstkontrolldatentabelle gespeichert sind. Der Ausgang der Modellgleichungs-Berechnungseinheit ist mit dem ersten Eingang der CTP-Summationsberechnungseinheit des CTP-Berechnungs-Moduls verbunden, in welcher die abschließende Berechnung der 1440 Tageseinzelwerte des 24-Stunden-Blutglukosekonzentrationsverlaufs x(t) eines Individuums für den Zeitraum von 0:00 Uhr bis 24:00 Uhr erfolgt.

Mittels der Insulinwirkprofil-Berechnungseinheit des CTP-Berechnungsmoduls wird das individuelle 24h-Insulinwirkprofil IE(t) eines Individuums unter Verwendung der Gleichungen (3.1) und (3.2), die in der Insulinwirkprofil-Berechnungseinheit abgelegt sind, berechnet. Die Berechnung erfolgt nach dem erfindungsgemäßen Verfahren im Minutenzeitraster, so dass am Ende der Berechnung 1440 Einzelwerte vorliegen, die auf die Tageszeitskala von 0:00 Uhr bis 24:00 Uhr abgebildet sind. Anschließend werden die der Tageszeit zugeordneten 1440 Einzelwerte des so berechneten 24-Stunden-Insulinwirkprofils IE(t) über den Ausgang der Insulinwirkprofil-Berechnungseinheit an den zweiten Eingang der CTP-Summations-Berechnungseinheit übergeben und in der CTP-Summations-Berechnungseinheit gespeichert. Die für die Berechnung des individuellen 24-Stunden-Insulinwirkprofils IE(t) erforderlichen Eingangsgrößen IE und $F_n$ werden vom Ausgang der Insulinapplikationskomponente des CTP-Eingangsgrößenmoduls, in welcher die Eingangsgrößen IE und $F_n$ dem der Insulinapplikationskomponente (IE, $F_n$) zugeordneten Sektoren der Selbstkontrolldatentabelle gespeichert sind, an den Eingang der 24-Stunden-Insulinwirkprofil-Berechnungseinheit des CTP-Berechnungsmoduls gelegt.

Die Berechnung des 24-Stunden-Nahrungsanflutungsprofils BE(t) erfolgt nach dem erfindungsgemäßen Verfahren in der BE-Anflutungs-Berechnungseinheit des CTP-Berechnungs-moduls im Minutenzeitraster, so dass am Ende des Berechnungsprozesses 1440 Einzelwerte ermittelt wurden, die nach Abbildung auf die Tageszeitachse über den Ausgang der BE-Anflutungs-Berechnungseinheit an den dritten Eingang der CTP-Summations-Berechnungseinheit geführt und anschließend in der CTP-Summations-Berechnungseinheit abgespeichert werden. Die für die Berechnung des individuellen BE-Anflutungsprofils erforderlichen BE-Eingangsgrößen werden aus der Nahrungsaufnahmekomponente des CTP-Eingangsgrößenmoduls, in welcher die Eingangsgröße BE in dem der Nahrungsaufnahmekomponente (BE) entsprechenden Sektor der Selbstkontrolldatentabelle gespeichert sind, über den Ausgang der Nahrungsaufnahmekomponente (BE) an den Eingang der BE-Anflutungs-Berechnungseinheit gelegt.

Die Berechnung der 24-Stunden-OAD-Wirkprofile OAD(t) erfolgt im Minutenzeitraster nach dem erfindungsgemäßen Verfahren in der OAD-Wirkprofil-Berechnungseinheit des CTP-Berechnungsmoduls, wobei die für die Berechnung erforderlichen OAD-Eingangsgrößen aus dem der Komponente Applikation oraler Antidiabetika des CTP-Eingangsgrößenmoduls zugeordneten Sektor der Selbstkontrolldatentabelle über den Ausgang der Applikation oraler Antidiabetika an den Eingang der OAD-Wirkprofil-Berechnungseinheit des CTP-Berechnungs-moduls gelegt werden. Die so berechneten 1440 Einzelwerte des 24-Stunden-OAD-Wirkprofils OAD(t) eines Individuums werden nach Abbildung auf die Tageszeitskala von 0:00 Uhr bis 24:00 Uhr über den Ausgang der OAD-Wirkprofil-Berechnungseinheit an den vierten Eingang der CTP-Summations-Berechnungseinheit des CTP-Berechnungsmoduls geführt und in der CTP-Summations-Berechnungseinheit gespeichert.

Die Berechnung der 24-Stunden-IDE-Wirkprofile IDE(t) körperlicher Aktivitäten wird nach dem erfindungsgemäßen Verfahren mittels der IDE-Wirkprofil-Berechnungseinheit des CTP-Berechnungsmoduls im Minutenzeitraster ausgeführt. Die für die Berechnung erforderlichen IDE-Eingangsgrößen werden hierzu aus dem der körperliche Aktivitätenkomponente des Eingangsgrößenmoduls zugeordneten Sektor der Selbstkontrolldatentabelle über den Ausgang der körperliche Aktivitätenkomponente an den Eingang der IDE-Wirkprofil-Berechnungseinheit des CTP-Berechnungsmoduls gelegt. Nach Berechnung der 1440 Tageseinzelwerte des 24-Stunden-IDE-Wirkprofils und deren Abbildung auf die Tageszeitskala von 0:00 Uhr bis 24:00 Uhr wird das so berechnete 24-Stunden-IDE-Wirkprofil IDE(t) über den Ausgang der IDE-Wirkprofil-Berechnungseinheit an den fünften Eingang der CTP-Summations-Berechnungseinheit des CTP-Berechnungsmoduls geführt und in der CTP-Summations-Berechnungseinheit gespeichert.

Mittels der Referenzblutglukosetagesprofil-Berechnungseinheit des CTP-Berechnungsmoduls werden nach dem erfindungsgemäßen Verfahren das Referenzblutgukosetagesprofil BG(t) und der Startwert x(0)t für die numerische Integration des Modellgleichungssystems erzeugt und über den Ausgang der Referenzblutglukosetagesprofil-Berechnungseinheit an den sechsten Eingang der CTP-Summations-Berechnungeinheit des CTP-Berechnungsmoduls geführt. Die für die Berechnung des Referenzblutglukosetagesprofils BG(t) und den Startwert x(0) erforderlichen Blutglukosemesswerte werden aus dem der Referenzblutglukosemesswertekomponente des CTP-Eingangsgrößenmoduls zugeordneten Sektor der Selbstkontrolldatentabelle vom Ausgang der Referenzblutglukosemesswertekomponente an den Eingang der Referenzblutglukosetagesprofil-Berechnungseinheit des CTP-Berechnungsmoduls übergeben.

Die Berechnung des individuellen 24-Stunden-Blutglukosekonzentrationsverlaufs x(t) eines Individuums erfolgt abschließend auf der Grundlage des erfindungsgemäßen Modellgleichungssystems mit den vier Zustandsgrößen Blutglukosekonzentrationsverlauf (dx/dt), endogene Glukosebilanz (du/dt), endogener Insulinkatabolismus (dy/dt) und Exercise induzierte Glukoseverwertung (de/dt) mittels der CTP-Summations-Berechnungseinheit. Hierzu werden zunächst die berechneten und in der CTP-Summations-Berechnungseinheit gespeicherten 24-Stunden-Wirk- und 24-Stunden-Anflutungsprofile IE(t), BE(t), OAD(t) und IDE(t) in die Modellgleichungen (1) bis (4) an entsprechender Stelle eingefügt. Anschließend wird das Modellgleichungssystem im Minutenzeitraster nach der Zustandgröße dx/dt durch numerische Integration mit dem Startwert x(0)t gelöst. Die Lösung liefert die gesuchten 1440 Einzelwerte des zu berechnenden 24-Stunden-Blutglukosekonzentrationsverlaufs x(t), welcher nach Abbildung auf die Tageszeitskala von 0:00 Uhr bis 24:00 Uhr in der CTP-Summations-Berechnungseinheit des CTP-Berechnungsmoduls abgespeichert wird.

Die Visualisierung der berechneten 24-Stunden-Profile des Blutglukosekonzentrationsverlaufs x(t), der Insulinwirkung IE(t), der BE-Anflutung BE(t), der Wirkung oraler Antidiabetika OAD(t) der insuliäquivalenten Wirkung körperlicher Aktivitäten IDE(t) und des Referenzblutglukoseverlaufs BG(t) erfolgt nach Zusammenführung aller 24-Stunden-Profile zu dem persönlichen charakteristischen Tagesprofil des individuellen Glukosemetabolismus eines Individuums mittels des erfindungsgemäßen CTP-Darstellungsmoduls. Hierzu werden die im CTP-Berechnungs-Modul gespeicherten 24-Stunden-Profile x(t), IE(t), BE(t), OAD(t), IDE(t) und BG(t) über den Ausgang der CTP-Summations-Berechnungseinheit B7 des CTP-Berechnungsmoduls an den Eingang des CTP-Darstellungsmoduls übergeben, dessen CTP-Darstellungseinheit (A1) das nach dem erfindungsgemäßen Verfahren berechnete charakteristische Tagesprofil des individuellen Glukosemetabolismus eine Individuums als den angestrebten Metabolischen Fingerabdruck darstellt.

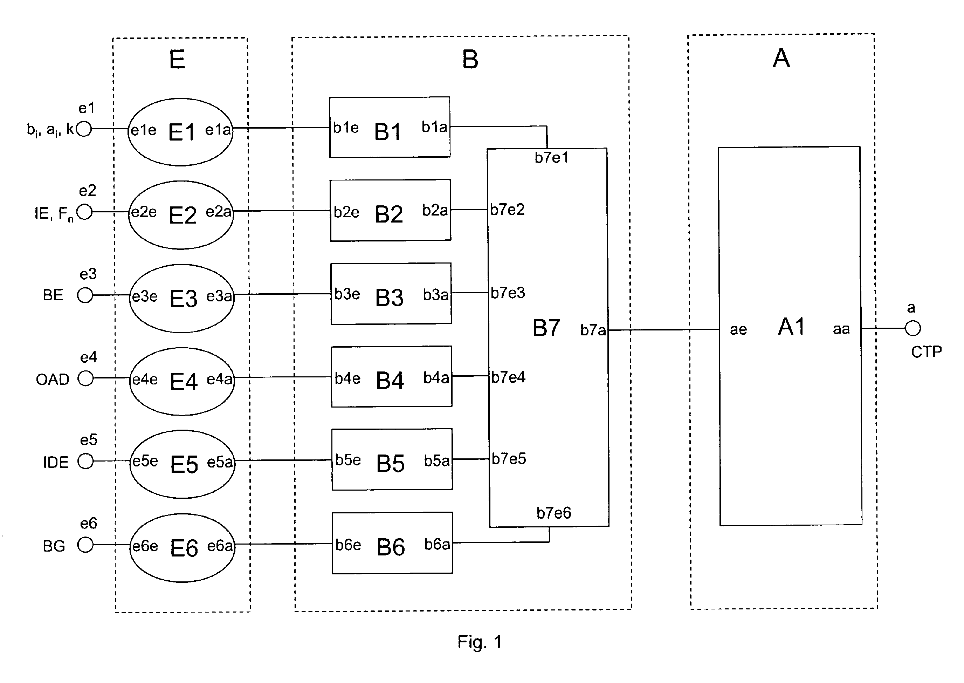

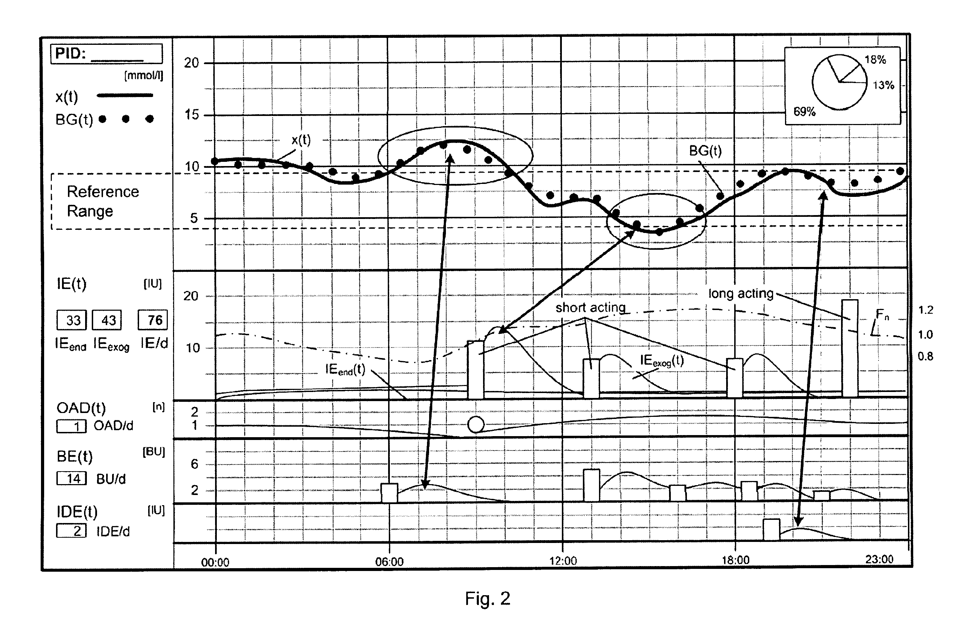

Nachstehend soll die Erfindung an einem Ausführungsbeispiel anhand der erfindungsgemäßen Anordnung gemäß Fig. 1 zur Durchführung des Verfahrens sowie anhand der beispielhaften grafischen Darstellung des persönlichen charakteristischen Tagesprofils des individuellen Glukosemetabolismus eines Individuums mit Typ-2-Diabetes gemäß Fig. 2 näher erläutert werden.

Die erfindungsgemäße Anordnung zur Bestimmung des persönlichen charakteristischen Tagesprofils des individuellen Glukosemetabolismus besteht gemäß Fig. 1 aus einem Mikrorechnersystem, das aus den drei Grundmodulen CTP-Eingangsgrößenmodul E, CTP-Berechnungsmodul B und CTP-Darstellungsmodul A modular aufgebaut ist.

Das CTP-Eingangsgrößenmodul E beinhaltet die Modellparameterkomponente E1 für die Parameterwerte $b_i$, $a_i$ und k, die Insulinapplikationskomponente E2 für IE und $F_n$, die Nahrungsaufnahmekomponente E3, die Komponente Applikation oraler Antidiabetika E4, die körperliche Aktivitätskomponente E5 und die Referenzblutglukosemesswertekomponente E6.

Das CTP-Berechnungsmodul B besteht aus der 24-Stunden-Modellgleichungs-Berechnungseinheit B1, der 24-Stunden-Insulinwirkprofil-Berechnungseinheit (IE(t)) B2, der 24-Stunden-BE-Anflutungs-Berechnungseinheit (BE(t)) B3, der 24-Stunden-OAD-Wirkprofil-Berechnungseinheit (OAD(t)) B4, der 24-Stunden-IDE-Wirkprofil-Berechnungseinheit (IDE(t)) B5, der Referenzblutglukosetagesprofil-Berechnungseinheit (BG(t)) B6 und der nachgeordneten CTP-Summations-Berechnungseinheit (x(t)) B7.

Das ausgangsseitig angeordnete CTP-Darstellungsmodul beinhaltet die Darstellungseinheit A für den Metabolischen Fingerabdruck.

An die sechs Eingabeschnittstellen e1 bis e6 des CTP-Eingangsgrößenmoduls E werden in chronologischer Reihenfolge an die Eingabeschnittstelle e1 die individualspezifischen Modellparameterwerte $b_i$, $a_i$, und k des erfindungsgemäßen Modellgleichungssystems mit den Gleichungen (1) bis (4) des mathematischen Modells zur Beschreibung des physiologischen Glukosemetabolismus, an die Eingabeschnittstelle e2 die typischerweise täglich vorgenommenen Insulinapplikationen IE sowie die F-Faktoren zur Beschreibung des individuellen Tagesgangs der Insulinwirkung $F_n$, an die Eingabeschnittstelle e3 die typischerweise täglich aufgenommenen Broteinheiten BE, an die Eingabeschnittstelle e4 die täglich oral verabfolgten Antidiabetika OAD, an die Eingabeschnittstelle e5 die typischerweise täglich ausgeführten sportlichen Aktivitäten IDE und an die Eingabeschnittstelle e6 die mittels kontinuierlichem Blutglukosemonitoring gemessenen Referenzblutglukosemesswerte BG des Individuums mit Typ-2-Diabetes beispielhaft gelegt.

Die individualspezifischen Parameterwerte $b_i$, $a_i$, und k des Modellgleichungssystems werden als numerische Werte vorzugsweise über die Tastatur der erfindungsgemäßen Mikrorechneranordnung eingegeben und an den Eingang e1e der Modellparameterkomponente E1 geführt, wo sie im entsprechenden Sektor der Selbstkontrolldatentabelle des CTP-Eingangsgrößenmoduls als Modellparameterdatensatz gespeichert werden. In Tab. 1 ist beispielhaft der Modellparameterdatensatz für das Individuum mit Typ-2-Diabetes wiedergegeben.

Tab. 1

| Modellparameter | Wert | Modellparameter | Wert | Modellparameter | Wert |
|---|---|---|---|---|---|
| $b_0$ | 2,8439 | $a_0$ | 0,5752 | k | 0,1470 |
| $b_{0k}$ | 0,6500 | $a_1$ | 0,0287 | | |
| $b_1$ | 0,0161 | $a_2$ | 0,0327 | | |
| $b_2$ | 0,0489 | $x_w$ | 5,0000 | | |
| $b_3$ | 0,0017 | | | | |

Über die Eingabeschnittstellen e2 bis e5 werden in chronologischer Folge die Daten der täglichen Insulinapplikationen IE, der täglich aufgenommen Nahrungseinheiten BE, die oral verabfolgten Antidiabetika OAD und der sportlichen Aktivitäten IDE des Individuums mit Typ-2-Diabetes eingegeben und den Eingängen e2e bis e5e der Komponenten E2 bis E5 des CTP-Eingangsgrößenmoduls E zugeführt sowie als Selbstkontrolldatensatz SK-Datensatz strukturiert in den entsprechenden Sektoren der Selbstkontrolldatentabelle des CTP-Eingangsgrößenmoduls E gespeichert. Die Eingabe der Selbstkontrolldaten kann sowohl über die Tastatur der erfindungsgemäßen Mikrorechneranordnung als auch über Schnittstellen zu bekannten elektronischen Datenmanagementsystemen erfolgen, soweit derartige Medien bei einem Individuum Anwendung finden.

Tab. 2 zeigt beispielhaft den strukturierten Selbstkontrolldatensatz des Individuums mit Typ-2-Diabetes, wie er in der Selbstkontrolldatentabelle des CTP-Eingangsgrößenmoduls E nach der Eingabe der Selbstkontrolldaten für die weitere Verarbeitung abgespeichert ist.

Tab. 2

| IE | | | BE | | OAD | | | IDE | | |
|---|---|---|---|---|---|---|---|---|---|---|
| Dosis | Art | Zeit | Menge | Zeit | Formulation | Dosis | Zeit | Intensität | Dauer | Zeit |
| 11 IE | Normal | 09:00 | 3 BE | 06:00 | Biguanid | 850 mg | 09:00 | mittel | 30 min | 19:00 |
| 7 IE | Normal | 13:00 | 5 BE | 13:00 | | | | | | |
| 7 IE | Normal | 18:00 | 2 BE | 16:00 | | | | | | |
| 18 IE | Basal | 22:00 | 3 BE | 18:30 | | | | | | |
| | | | 1 BE | 21:00 | | | | | | |

Die beispielhafte Eingabe der 24 Faktoren $F_1$ bis $F_{24}$ ($F_n$) zur Beschreibung des individualspezifischen Tagesgangs der Insulinwirkung des Individuums mit Typ-2-Diabetes erfolgt in elektronischer Form über die Eingabeschnittstelle e2, von wo aus sie zum Eingang e2e der Insulinapplikationskomponente E2 des CTP-Eingangsgrößenmoduls E geführt und dort im entsprechenden Sektor der Selbstkontrolldatentabelle im äquidistanten zeitlichen Abstand von 1 Stunde, beginnend mit dem Zeitintervall von 0:00 Uhr bis 01:00 Uhr und endend mit dem Zeitintervall von 23:00 Uhr bis 24:00 Uhr abgespeichert werden.

In Tab. 3 sind die individualspezifischen Faktoren $F_n$ des Tagesgangs der Insulinwirkung eines Individuums mit Typ-2-Diabetes beispielhaft aufgelistet.

Tab. 3

| Faktor | $F_1$ | $F_2$ | $F_3$ | $F_4$ | $F_5$ | $F_6$ | $F_7$ | $F_8$ | $F_9$ | $F_{10}$ | $F_{11}$ | $F_{12}$ | $F_{13}$ | $F_{14}$ | $F_{15}$ | $F_{16}$ | $F_{17}$ | $F_{18}$ | $F_{19}$ | $F_{20}$ | $F_{21}$ | $F_{22}$ | $F_{23}$ | $F_{24}$ |
|---|---|---|---|---|---|---|---|---|---|---|---|---|---|---|---|---|---|---|---|---|---|---|---|---|
| Wert | 1,0 | 1,0 | 0,9 | 0,8 | 0,8 | 0,7 | 0,7 | 0,8 | 0,8 | 0,9 | 0,9 | 1,0 | 1,1 | 1,1 | 1,2 | 1,2 | 1,3 | 1,3 | 1,3 | 1,2 | 1,2 | 1,1 | 1,1 | 1,0 |

Die Eingabe der gemessenen Blutglukosekonzentrationsreferenzwerte BG, die bei dem beispielhaft gewählten Individuum mit Typ-2-Diabetes kontinuierlich im Zeitabstand von 5 Minuten gemessen und in einem handelsüblichen Monitor gespeichert sind, erfolgt durch Auslesen der gespeicherten Monitordaten über die Eingabeschnittstelle e6 von wo aus sie dem Eingang e6e der Referenzblutglukosewertekomponente E6 des CTP-Eingangsgrößenmoduls E zugeführt und dort in Form von 288 Einzelwerten im äquidistanten Zeitabstand von 5 Minuten, beginnend mit dem Zeitintervall von 00:00 Uhr bis 00:05 Uhr und endend mit dem Zeitintervall von 23:55 Uhr bis 24:00 Uhr im entsprechenden Sektor der Selbstkontrolldatentabelle des CTP-Eingangsgrößenmoduls E gespeichert werden.

Nachdem der Modellparameterdatensatz, der Selbstkontrolldatensatz, die individualspezifischen Faktoren $F_n$ und die Blutglukosekonzentrationsreferenzwerte BG des beispielhaft gewählten Individuums mit Typ-2-Diabetes in der Selbstkontrolldatentabelle des CTP-Eingangsgrößen-Moduls E eingetragen worden sind, werden die Daten an das erfindungsgemäße CTP-Berechnungsmodul B mit den Berechnungseinheiten B1 bis B7 zur Berechnung des persönlichen CTP des Individuums mit Typ-2-Diabetes übergeben, wobei die Modelparameterwerte $b_i$, $a_i$ und k vom Ausgang e1a der Modellparameterkomponente E1 des CTP-Eingangsgrößen-Moduls E an den Eingang b1e der 24-Stunden-Modellgleichungs-Berechnungseinheit B1 des CTP-Berechnungsmoduls B, die Selbstkontrolldaten IE, BE, OAD und IDE über die Ausgänge e2a bis e5a der Komponenten E2 bis E5 des CTP-Eingangsgrößenmoduls E an die Eingänge b2e bis b5e der Berechnungseinheiten B2 bis B5 des CTP-Berechnungsmoduls B, die Faktoren $F_n$ des Tagesgangs der Insulinwirkung über den Ausgang e2a der Insulinapplikationskomponente E2 des CTP-Eingangsgrößenmoduls E an den Eingang b2e der 24-Stunden-Insulinwirkprofil-Berechnungseinheit B2 des CTP-Berechnungsmoduls B und die Blutglukosekonzentrationsreferenzwerte BG über den Ausgang e64 der Referenzblutglukosemesswertekomponente E6 des CTP-Eingangsgrößen-Moduls E an den Eingang b6e der Referenzblutglukosetagesprofil-Berechnungseinheit B6 des CTP-Berechnungsmoduls geführt werden. Anschließend erfolgt die Berechnung des persönlichen charakteristischen Tagesprofils des individuellen Glukosemetabolismus des Individuums mit Typ-2-Diabetes gemäß dem erfindungsgemäßen Verfahren auf der Grundlage des Modellgleichungssystems

Blutglukosekonzentrationsverlauf dx/dt gemäß Gleichung (1)
endogene Glukosebilanz du/dt gemäß Gleichung (2)
endogener Insulinmetabolismus dy/dt gemäß Gleichung (3)
Exercise induzierte Glukoseverwertung gemäß Gleichung (4)

mittels der Berechnungseinheiten B1 bis B7 des CTP-Berechnungsmoduls B.

Im Ergebnisse der Berechnungen liegt im CTP-Berechnungsmodul B erfindungsgemäß das persönliche charakteristische Tagesprofil des individuellen Glukosemetabolismus des beispielhaft gewählten Individuums mit Typ-2-Diabetes mit seinem 24-Stunden-Blutglukosekonzentrationsverlauf x(t) in Relation zu seinem 24-Stunden-Insulinwirkprofil IE(t), seinem 24-Stunden-Nahrungsanflutungsprofil BE(t), seinem 24-Stunden-OAD-Wirkprofil OAD(t), seinem 24-Stunden-IDE-Wirkprofil IDE(t) und seinem gemessenen Referenzblutglukosetagesprofil BG(t) vor, welches zur Visualisierung über den Ausgang b7a der CTP-Summations-Berechnungseinheit B7 des CTP-Berechnungsmoduls B an den Eingang ae der CTP-Darstellungseinheit A1 des CTP-Darstellungsmoduls A übergeben wird.

Die Visualisierung des ermittelten persönlichen CTP des Individuums mit Typ-2-Diabetes erfolgt in der Qualität eines Metabolischen Fingerabdrucks erfindungsgemäß durch die grafische Darstellung des charakteristischen Tagesprofils der Blutglukoskonzentration mit seinen Blutzucker senkenden und Blutzucker steigernden Einflußgrößen gemäß Fig. 2 mittels der CTP-Darstellungseinheit A1 des CTP-Darstellungsmoduls A.

In Fig. 2 ist das charakteristische Tagesprofil des individuellen Glukosemetabolismus mit seinen Blutzucker senkenden und Blutzucker steigernden Einflussgrößen eines Individuums mit Typ-2-Diabetes als Metabolischer Fingerabdruck beispielsweise dargestellt.

Über dem Tageszeitfenster von 00:00 Uhr bis 24:00 Uhr sind im oberen Teil der erfindungsgemäßen Visualisierungsgrafik gemäß Fig. 2 der auf der Grundlage des mathematischen Modells des physiologischen Glukosemetabolismus mit den Modellgleichungen (1) bis (4) berechnete individuelle 24-Stunden-Blutglukosekonzentrationsverlauf x(t) des Individuums mit Typ-2-Diabetes als ausgezogene Linie und sein gemessenes Referenzblutglukosetagesprofil BG (t) als gepunktete Linie im Vergleich zu dem markierten Referenzbereich des Blutglukosetagesprofils, welcher bei einer angemessenen Stoffwechselführung weder unterschritten noch überschritten werden sollte, dargestellt. Über- oder/und Unterschreitungen des Referenzbereichs des Blutglukosetagesprofils werden als Tortengrafik in Form eines Qualitätsauges in Prozent der Glukosewerte, die außerhalb des Referenzbereiches liegen angegeben, wobei 100 % bedeutet, dass sich alle Blutglukosewerte innerhalb des Referenzbereiches befinden. Das beispielhaft berechnete Qualitätsauge für das Individuum mit Typ-2-Diabets ist in der oberen rechten Ecke der Visualisierungsgrafik angeordnet.

Unterhalb der Darstellung der berechneten (x(t)) und gemessenen (BG(t)) Tagesprofile der Glukosekonzentration sind für das Individuum mit Typ-2-Diabetes in chronologischer Folge seine erfindungsgemäß berechneten 24-Stunden-Wirk- und 24-Stunden-Anflutungsprofile für Insulin IE(t), für die Nahrungsaufnahme BE(t), für die Verabfolgung oraler Antidiabetika OAD(t) und für körperliche Aktivitäten IDE(t) dargestellt, die entsprechend den für das Individuum mit Typ-2-Diabetes zutreffen Angaben zum Selbstkontrolldatensatz gemäß Tab. 2 auf der 24-Stunden-Tageszeitskala gemäß der im erfindungsgemäßen Verfahren beschriebenen Darstellungsweise angeordnet. In der Insulinanflutungsgrafik sind zudem die in Tab. 3 aufgelisteten individualspezifischen Faktoren $F_n$ zur Beschreibung des Tagesgangs der Insulinwirkung des Individuums mit Typ-2-Diabetes in Form einer gestrichelten Linie grafisch veranschaulicht.

Die Kenntlichmachung der ursächlichen Zusammenhange zwischen den markierten Bereichen in dem berechneten 24-Stunden-Blutglukosekonzentrationsverlauf x(t) und den berechneten 24-Stunden-Profilen der Insulinwirkung IE(t), der Nahrungsanflutung BE(t), der Wirkung oraler Antidiabetika OAD(t) und der insulinäquivalenten Wirkung körperlicher Aktivitäten IDE(t) des persönlichen CTP des Individuums mit Typ-2-Diabetes erfolgt erfindungsgemäß mittels der in Fig. 2 entsprechend eingeblendeten Beziehungspfeile.

## Patentansprüche

1. Verfahren zur rechnergestützten Bestimmung des charakteristischen Tagesprofils des individuellen Glukosemetabolismus unter Anwendung des mathematischen Modells zur Beschreibung des physiologischen Glukosemetabolismus mit den vier Zustandsgrößen Blutglukosekonzentrationsverlauf dx/dt, endogene Glukosebilanz du/dt, endogener Insulinmetabolismus dy/dt und Exercise induzierte Glukoseverwertung de/dt, **dadurch gekennzeichnet, dass** aus vorhandenen Selbstkontrollangaben eines Individuums zu seinen typischerweise täglich aufgenommenen Broteinheiten (BE), seinen Medikationen mit oral verabfolgten Antidiabetika unterschiedlicher Wirkklassen ($OAD_i$) und Insulin (IE) sowie seinen sportlichen Aktivitäten (IDE) der persönliche tägliche Blutglukosekonzentrationsverlauf x(t) dieses Individuums in Relation zu den endogenen Bilanzgrößen Glukosebilanz u(t) und Insulinmetabolismus y(t) sowie der Exercise induzierten Glukoseverwertung e(t) mittels eines Mikrorechnersystems berechnet wird und dass die im Minutentakt berechneten Werte des täglichen Blutglukosekonzentrationsverlaufs x(t) des Individuums sequenziell auf einer 24-Stunden-Tagesskala abgebildet werden, dass danach das persönliche 24-Stunden-Insulinwirkprofil IE(t) für die Eingangsgröße Insulinzufuhr (IE), das persönliche 24-Stunden-BE-Anflutungsprofil BE(t) für die Eingangsgröße Kalorienzufuhr (BE), das persönliche 24-Stunden-OAD-Wirkprofil OAD(t) für die Eingangsgröße Applikation oraler Antidiabetika (OAD) und das persönliche 24-Stunden-IDE-Wirkprofil (IDE(t)) für die Eingangsgröße körperliche Aktivität (IDE) für ein Individuum bestimmt werden, dass danach ein individualspezifisches Referenzblutglukosetagesprofil BG(t) aus gemessenen Blutkosewerten zur Validitätsprüfung des modellgestützt berechneten persönlichen Blutglukosekonzentrationsverlaufs x(t) eines Individuums generiert und der Startwert x(0)t für das numerische Integrationsverfahren erzeugt wird, dass die Generierung des Referenzblutglukosetagesprofils BG(t) **dadurch** erreicht wird, dass die Blutglukosereferenzwerte $BG_n$ eines Individuums vorzugsweise quasikontinuierlich in einem 5 Minuten-Zeitraster über einen Zeitraum von bis zu 72 Stunden gemessen und aufgezeichnet werden, dass aus den aufgezeichneten Einzelmesswerten anschließend unter Anwendung bekannter Verfahren der mathematischen Statistik und Mittelwertsbildung das Referenzblutglukosetagesprofil BG(t) mit einer Vielzahl von Messpunkten erzeugt und in äquidistanten Zeitabständen von 5 Minuten auf der 24-Stunden-Tageszeitskala abgebildet wird, dass die Generierung des Startwertes x(0)t durch Berechnung des Tagesmittelwertes des Refenzblutglukosetagesprofils gemäß der bekannten mathematischen Beziehung $BG_{mittel}$ = Summe $BG_n$/n erfolgt, wobei der berechnete Mittelwert der Tagesblutglukosekonzentration gleich dem Startwert x(0)t für das numerische Integrationsverfahren zur modellgestützte Berechnung des individualspezifischen Blutglukosekonzentrationsverlaufs x(t) eines Individuums gesetzt wird und dass die 24-Stunden-Profile des modellgestützt berechneten Blutglukosekonzentrationsverlaufs x(t), der Insulinwirkung IE(t), der BE-Anflutung BE(t), der Wirkung oraler Antidiabetika OAD(t), der körperlichen Aktivitäten IDE(t) und des gemessenen Referenzblutglukosetagesprofils BG(t) eines Individuums über der 24-Stunden-Tageszeitskala unter Verwendung der entsprechenden Angaben aus der Selbstkontrolldatentabelle synchronisiert und zu dem persönlichen charakteristischen Tagesprofil dieses Individuums in der Qualität eines Metabolischen Fingerabdrucks seines Glukosmetabolismus zusammengeführt und in einer Ausgabedatei zur weiteren Verwendung gespeichert werden und dass die insgesamt pro Tag verabreichten Insulineinheiten IE/Tag mit differenziertem Ausweis für den endogenen und den exogen Anteil durch Integration der jeweiligen Insulinwirkflächen, die pro Tag aufgenommenen BE/Tag durch Integration der BE-Anflutungsfläche, der Anzahl von OAD-Tabletten/Tag durch Integration der OAD-Wirkflächen sowie die Gesamtwirkung körperlicher Aktivitäten IDE/Tag durch Integration der IDE-Wirkfläche ermittelt und gleichfalls in der Ausgabedatei gespeichert werden und dass das persönliche charakteristischen Tagesprofil dieses Individuums in Form des individuellen metabolischen Fingerabdrucks seines Glukosemetabolismus mittels bekannter Methoden der grafischen Präsentation visualisiert wird, wobei die sechs 24-Stunden-Profile x(t), IE(t), BE(t), OAD(t), IDE(t) und BG(t) des in der Ausgabedatei gespeicherten persönlichen charakteristischen Tagesprofils dieses Individuums über einer einheitlichen 24-Stunden-Tageszeitskala gemäß ihrer jeweiligen spezifischen quantitativen und qualitativen Eigenschaften grafisch dargestellt werden.

**2.** Verfahren zur rechnergestützten Bestimmung des charakteristischen Tagesprofils des individuellen Glukosemetabolismus nach Anspruch 1, **dadurch gekennzeichnet, dass** die individualspezifische Parameterwerte des mathematischen Modells des physiologischen Glukosemetabolismus mit den Zustandgrößen Blutglukosekonzentrationsverlauf = $dx/dt = u(t) + (BE(t) - OAD_1(t))$, endogene Glukosebilanz = $du/dt = - (b_1+b_2)u(t) - b_3(y(t) + e(t) + OAD_2(t))$ $+ b_1(b_0 - b_{0k}(BE(t)) - BE(t) + OAD_3(t))$, endogener Insulinmetabolismus = $dy/dt = -k(y(t)) + [a_0 + a_1(x(t) - x_w) + a_2(BE(t)/dt)] + OAD_4(t) + IE_{exog}(t)$ und Exercise induzierte Glukoseverwertung = $de/dt = -k(e(t)) + IDE(t)$ kausal den metabolischen Vorgängen

$b_0$ = endogener Glukosezustrom,
$b_{0k}$ = Wirkfaktor für die BE-abhängige Verminderung des endogenen Glukosezustroms,
$b_1$ = Verstärkungsfaktor für den endogenen Glukoseumsatz,
$b_2$ = Zeitkonstante für den endogenen Glukoseumsatz,
$b_3$ = Wirkfaktor für die insulingetriggerte Glukoseverwertung,
$a_0$ = glukoseunabhängige endogene Insulinbereitstellung,
$a_1$ = Wirkfaktor für die glukosegesteuerte endogene Insulinbereitstellung,
$a_2$ = Wirkfaktor für die BE-gesteuerte endogene Insulinbereitstellung,
$x_w$ = Glukosekonzentration, bei der die endogene Insulinbereitstellung zu Null wird, und
$k$ = Wirkfaktor für den Insulinkatabolismus

zugeordnet sind und gemeinsam mit den Faktoren $F_n$ eines Individuums iterativ mittels bekannter mathematischer Optimierungsverfahren bestimmt werden.

**3.** Verfahren zur rechnergestützten Bestimmung des charakteristischen Tagesprofils des individuellen Glukosemetabolismus nach Anspruch 1, **dadurch gekennzeichnet, dass** für die Berechnung des persönlichen charakteristischen Tagesprofils die Insulinzufuhr IE, welche sich aus einem exogenen zuzuführenden körperfremden Anteil $IE_{exog}(t)$ und einem körpereigenen zuzuführenden endogenen Anteil = $IB_{endog}(t) = [a_0 + a_1x(t) - x_w) + a_2(BE(t)/dt)]$ additiv zu der Gesamtinsulinzufuhr = $IE(t) = [IE_{exog}(t) + IE_{endog}(t)] F_n(t)$ zusammensetzt, mit den individualspezifischen Faktoren $F_n(t)$ zur Berücksichtigung des individuellen Tagesgangs der Insulinwirkung multipliziert wird.

**4.** Verfahren zur rechnergestützten Bestimmung des charakteristischen Tagesprofils des individuellen Glukosemetabolismus nach Anspruch 1, **dadurch gekennzeichnet, dass** zur rechnergestützten Bestimmung des persönlichen 24-Stunden-Insulinwirkprofils IE(t) zuerst quasi kontinuierlich im Minutentakt der körpereigene Anteil $IE_{endog}(t)$ gemäß der Gleichung $IE_{endog}(t) = [a_0 + a_1x(t) - x_w) + a_2(BE(t)/dt)]$ berechnet und auf die 24-Stunden-Tagesskala abgebildet wird und dass die Berechnung des 24-Stunden-Insulinwirkprofils des exogenen Anteils $IE_{exog}(t)$ erfolgt, indem die individuellen Angaben zur Applikation körperfremden Insulins nach Tageszeit, Insulinart und Dosis des betreffenden Individuums in eine im Rechner angelegte Selbstkontrolldatentabelle eingetragen und gespeichert werden, die Angaben zur Insulinart mit einer im Rechner gespeicherten Insulintabelle, in der alle handelsüblichen Insulinpräparate nach den Wirkklassen kurzwirksame Insuline, Normalinsuline, Semilenteinsuline, Basalinsuline und Mischinsuline geordnet abgelegt sind und mit Ausnahme der Mischinsuline jeder Wirkklasse ein normiertes Grundwirkungsprofil für 1 IE mit den Kernangaben zu Wirkungsstärke und Wirkungsdauer zugeordnet ist, welche gleichfalls in der Insulintabelle abgelegt sind, verglichen werden und dass bei Übereinstimmung zwischen einer in der Selbstkontrolldatendatei abgelegten Insulinart und einem Insulinpräparat aus einer der fünf Wirkklassen in der Insulintabelle die entsprechenden Angaben für dieses Insulinpräparat aus der Insulintabelle selektiert und im Rechner zwischengespeichert werden und dass nach Identifizierung aller exogen verabfolgten Insulinpräparate die multiplikative Hochrechnung des Grundprofils von 1 IE flächenäquidistant vorgenommen wird, wobei der jeweilige Dosismultiplikator zu jeweils 50% auf die Wirkungsstärke und auf die Wirkungsdauer verteilt wird, und auf die tatsächlich verabfolgte Dosis gemäß den Angaben aus der Selbstkontrolldatentabelle erfolgt und dass die so berechneten und zwischengespeicherten Einzelinsulinwirkprofile entsprechend den aus der Selbstkontrolldatentabelle zu entnehmenden Verabfolgungszeiten zu einem 24-Stunden-Insulinwirkprofil $IE_{exog}(t)$ für den exogenen Anteil am gesamten 24-Stunden-Insulinwirkprofil IE(t) des persönlichen charakteristischen Tagesprofils des individuellen Glukosemetabolismus eines Individuums zusammengeführt werden und dass auf das gemäß der Gleichung Gesamtinsulinzufuhr = $IE(t) = [IE_{exog}(t) + IE_{endog}(t)] F_n(t)$ additiv zu einem Gesamtinsulinwirkprofil $IE(t) = IE_{endog}(t) + IE_{exog}(t)$ zusammengeführte 24-Stunden-Insulinwirkprofil IE(t) der zuvor für jedes Individuum ermittelte, individualspezifische Tagesgang in der Insulinwirkung aufmultipliziert wird, wobei der individualspezifische Tagesgang der Insulinwirkung in Form von 24 $F_n$ - Multiplikationsfaktoren $F_1$ bis $F_{24}$, die die Veränderungen in der Insulinwirkung pro Stunde über den 24 Stunden-Tag hinweg beschreiben, für jedes Individuum im Rechner in einem gesonderten Sektor der Selbstkontrolldatentabelle abgespeichert ist.

**5.** Verfahren zur rechnergestützten Bestimmung des charakteristischen Tagesprofils des individuellen Glukosemetabolismus nach Anspruch 1, **dadurch gekennzeichnet, dass** die Berechnung des 24-Stunden-BE-Anflutungsprofils BE(t) eines Individuums **dadurch** erfolgt, dass die individuellen Angaben zur BE-Aufnahme hinsichtlich der Menge der zu den jeweiligen Mahlzeiten aufgenommenen BE und zu den Zeitpunkten der BE-Aufnahmen in die Selbstkontrolldatentabelle eingetragen und dort gespeichert werden, dass das der jeweiligen Nahrungsaufnahe zugeordnete BE-Anflutungsprofil in Relation zu einem in der BE-Tabelle abgespeicherten normierten Anflutungsgrundprofil für 1 BE mit Angaben zur Anflutungshöhe und zur Anflutungsdauer berechnet wird und dass die Hochrechnung von dem 1-BE-Anflutungsgrundprofil auf das Profil, welches den tatsächlich aufgenommenen BE entspricht, analog zu den Insulinwirkprofilen flächenäquidistant erfolgt und dass danach die so berechneten einzelnen BE-Anflutungsprofile schließlich zu dem 24-Stunden-BE-Anflutungsprofil BE(t) im persönlichen charakteristischen Tagesprofil eines Individuums zusammengeführt und entsprechend den zeitlichen Angaben aus der Selbstkontrolldatentabelle auf die 24-Stunden-Tagesskala abgebildet werden.

**6.** Verfahren zur rechnergestützten Bestimmung des charakteristischen Tagesprofils des individuellen Glukosemetabolismus nach Anspruch 1, **dadurch gekennzeichnet, dass** die Berechnung des persönlichen 24-Stunden-OAD-Wirkprofils OAD(t) oral aufgenommener Antidiabetika OAD eines Individuums **dadurch** erfolgt, dass die individuellen Angaben zur Applikation oral verabfolgter Antidiabetika nach Tageszeit, OAD-Formulation und verabreichter Dosis des betreffenden Individuums in der Selbstkontrolldatentabelle erfasst und gespeichert werden, dass die Angaben zur OAD-Formulation und zur verabreichten Dosis mit einer im Mikrorechner gespeicherten OAD-Tabelle, in der alle handelsüblichen oralen Antidiabetika differenziert nach den Wirkklassen Alphaglukosidasehemmer, Insulinsensitizer, Biguanide, Sulphonylharnstoffe und Glinide abgelegt sind und jedem der Wirkklassen auf die jeweilige Basisdosis normierte Grundwirkprofile mit Angaben zur Wirkstärke und zur Wirkdauer zugeordnet sind, verglichen werden und dass bei Übereinstimmung zwischen einem der angegebenen OAD-Formulationen mit einem OAD-Präparat aus der OAD-Tabelle das entsprechende, dosisbezogene Wirkprofil dieses OAD-Präparats aus der Tabelle selektiert und zur Abbildung der einzelnen OAD-Wirkprofile entsprechend den zeitlichen Angaben aus der Selbstkontrolldatentabelle auf die 24-Stunden-Tagesskale bereitgestellt wird, wodurch über den Tag hinweg das OAD-Gesamtwirkprofil OAD(t) des persönlichen charakteristischen Tagesprofil entsteht.

**7.** Verfahren zur rechnergestützten Bestimmung des charakteristischen Tagesprofils des individuellen Glukosemetabolismus nach Anspruch 1, **dadurch gekennzeichnet, dass** das individuelle 24-Stunden-Insulin-Dose-Equivalent-Wirkprofil (IDE(t)) in Form von individualspezifischen Insulinwirkäquivalenten körperlicher Aktivitäten berechnet wird **dadurch**, dass die individuellen Angaben zu körperlichen Aktivitäten in der Selbstkontrolldatentabelle nach Zeitpunkt, Art, Intensität und Dauer erfasst und gespeichert werden, dass diese Angaben auf ein IDE-Standardprofil mit der Wirkung von einer IE normiert, um aus diesen Angaben individualspezifische Insulinwirkäquivalente (IDE) in Form von Insulineinheiten (IE) und deren Wirkprofile (IDE(t)) berechnen zu können, wobei hierzu in einer IDE-Tabelle die drei Arten körperlicher Aktivitäten Laufen, Schwimmen und Radfahren und drei Intensitätsgrade leicht, mittel und schwer zur Auswahl zur Verfügung stehen und dass jeder Art körperlicher Aktivität und jedem Intensitätsgrad Multiplikationsfaktoren zugeordnet sind, welche zusammengenommen die Wirkstärke des gesuchten IDE-Profils bestimmen, wobei das Integral der zwischen Wirkstärke und gewählter Wirkdauer aufgespannten Wirkfläche dem für die ausgewählte körperliche Aktivität gesuchten IDE-Wirkprofil (IDE(t)) entspricht und dass die so berechneten IDE-Einzelwirkprofile entsprechend den aus der Selbstkontrolldatentabelle zu entnehmenden Zeitpunkten körperlicher Aktivitäten entlang der 24-Stunden-Tagesskala zu dem 24-Stunden-IDE-Wirkprofil (IDE(t)) des persönlichen charakteristischen Tagesprofil eines Individuums zusammengeführt werden.

**8.** Verfahren zur rechnergestützten Bestimmung des charakteristischen Tagesprofils des individuellen Glukosemetabolismus nach Anspruch 1 bis 7, **dadurch gekennzeichnet, dass** die Darstellung des persönlichen charakteristischen Tagesprofils eines Individuums in Form eines individuellen Metabolischen Fingerabdrucks seines Glukosemetabolismus in der Sequenz Blutglukosekonzentrationen, Insulinwirkprofile, BE-Anflutungsprofile, OAD-Wirkprofile, IDE-Wirkprofile, in einer 24-Stunden-Tagesskala erfolgt, dass der modellgestützt berechnete 24-Stunden-Blutglukosekonzentrationsverlauf x(t) und das gemessene Referenzblutglukosetagesprofil (BG(t)) des persönlichen charakteristischen Tagesprofils eines Individuums zusammen in dem dafür vorgesehenen Grafikteil als Konzentrationsverläufe wahlweise in der Dimension mmol/l oder mg/dl in der Form einer ersten Linie (x(t)) sowie einer zweiten Linie (BG(t)) dargestellt wird, dass in diesem Grafikteil ein Blutglukosekonzentrationsreferenzbereich eingeblendet wird, welcher den normalerweise üblichen Konzentrationsbereich der Blutglukose bei stoffwechselgesunden Individuen über den Tag hinweg aufzeigt, dass die prozentualen, auf den Tag bezogenen Anteile der modellgestützt berechneten Blutglukosekonzentrationswerte x(t), die den Referenzbereich unter- oder überschreiten, mittels einer geeigneten Grafik im Sinne eines Qualitätsauges in der rechten oberen Ecke des persönlichen charakteristischen Tagesprofil eines Individuums gesondert dargestellt werden, dass die Insulinwirkprofile (IE(t)) des persönlichen charakteristischen Tagesprofil eines Individuums additiv für die beiden Wirkprofile $IE_{endog}(t)$ und $IE_{exog}(t)$ in der Dimension Insulineinheiten grafisch dargestellt werden, wobei zur besseren Differenzierung für die Darstellung der beiden Insulinwirkprofilanteile $IE_{endog}(t)$ und $IE_{exog}(t)$ differenziert ausgewiesen werden, dass zu dem Wirkprofil $IE_{exog}(t)$ zu den Applikationszeiten jeweils erste schmale Rechtecksignale eingeblendet werden, deren Flächengestaltung mit der Insulinwirkklasse und deren Höhe mit der Anzahl der verabreichten Insulineinheiten korrespondieren, dass diesem Grafikteil auch die gesamten pro Tag verabfolgten IE, differenziert nach exogenem und endogenem Insulin, in numerischer Form aufgeführt, zugeordnet werden dass die grafische Darstellung der BE-Anflutungsprofile (BE(t)) in der Dimension Broteinheiten (BE) erfolgt, dass an den Zeitpunkten im Tagesablauf, an welchen gegessen wird, d.h. zu den Zeitpunkten, bei denen die jeweilige BE-Anflutung beginnt, zweite schmale Rechtecksignale eingeblendet werden, deren Höhe der Anzahl der aufgenommenen BE entspricht, dass diesem Grafikteil zugeordnet die insgesamt pro Tag aufgenommenen BE in numerischer Form eingeblendet werden, dass das OAD-Anflutungsprofil (OAD(t)) des persönlichen charakteristischen Tagesprofils eines Individuums analog dem BE-Anflutungsprofil (BE(t)) dargestellt wird, wobei die Dimension der grafischen Darstellung des OAD-Anflutungsprofils (OAD(t)) der Anzahl der eingenommenen Tabletten entspricht, dass anstelle der zweiten schmalen Rechtecksignale in der grafischen Darstellung des BE-Anflutungsprofils in der grafischen Darstellung des OAD-Anflutungsprofils (OAD(t)) zu den Zeiten der Tabletteneinnahme symbolhaft markante Signale angeordnet sind, deren Anzahl der Anzahl der eingenommenen Tabletten entspricht, dass zur Differenzierung der unterschiedlichen OAD-Wirkklassen jeder Wirkklasse eine dritte Kennzeichnung zugeordnet wird, dass für die grafische Darstellung des Wirkprofils körperlicher Aktivitäten in der Dimension Insulineinheitenäquivalente (IDE) im persönlichen charakteristischen Tagesprofil eines Individuums zu dem eigentlichen IDE-Wirkprofil (IDE(t)) zu den Zeitpunkten der körperlichen Aktivitäten zusätzlich ein drittes Rechtecksignal eingeblendet, dessen Höhe der Intensität und dessen Breite der Dauer der jeweiligen körperlichen Aktivität entspricht und zudem die insgesamt über den Tag hinweg wirksamen Insulinwirkäquivalente diesem Grafikteil in numerischer Form zugeordnet dargestellt werden und dass zur Visualisierung und Kenntlichmachung, welche ursächlichen Zusammenhänge zwischen den berechneten und dargestellten 24-Stunden-Wirk- und Anflutungsprofilen (IE(t)), (BE(t)), (OAD(t)) und (IDE(t)) und dem 24-Stunden-lutglukosekonzentrationsverlauf x(t) des persönlichen charakteristischen Tagesprofils eines Individuums bestehen, zwischen den Wirk- und Anflutungsprofilen und den entsprechend markierten Bereichen im Blutglukosekonzentrationsverlauf, für die ein derartiger kausaler Zusammenhang besteht, Beziehungspfeile in die grafische Darstellung des persönlichen charakteristischen Tagesprofils eingeblendet werden.

**9.** Anordnung zur rechnergestützten Bestimmung des charakteristischen Tagesprofils (CTP) des individuellen Glukosemetabolismus in Form eines Metabolischen Fingerabdrucks, **dadurch gekennzeichnet, dass** sie aus einem Mikrorechnersystem mit einem CTP-Eingangsgrößenmodul (E) mit vorgeschalteten Eingabeschnittstellen zur Übernahme der Selbstkontrolldaten als Eingabegrößen, einem nachgeordneten CTP-Berechnungsmodul (B) mit angeschlossenen CTP-Darstellungsmodul (A) besteht, dass in chronologischer Reihenfolge an die ersten Eingabeschnittstelle (e1) die individualspezifischen Modellparameterwerte $b_i$, $a_i$, und k des Modellgleichungssystems mit den Gleichungen (1) bis (4) des mathematischen Modells zur Beschreibung des physiologischen Glukosemetabolismus mit den vier Zustandsgrößen Blutglukosekonzentrationsverlauf (dx/dt), endogene Glukosebilanz (du/dt), Insulinmetabolismus, (dy/dt) und Exercise induzierte Glukoseverwertung (de/dt), an die zweite Eingabeschnittstelle (e2) die typischerweise täglich vorgenommenen Insulinapplikationen (IE) sowie die F-Faktoren zur Beschreibung des individuellen Tagesgangs der Insulinwirkung $(F_n)$, an die dritte Eingabeschnittstelle (e3) die typischerweise täglich aufgenommenen Broteinheiten (BE), an die vierte Eingabeschnittstelle (e4) die täglich oral verabfolgten Antidiabetika (OAD), an die fünfte Eingabeschnittstelle (e5) die typischerweise täglich ausgeführten sportlichen Aktivitäten (IDE) und an die Eingabeschnittstelle e6 die mittels kontinuierlichem Blutglukosemonitoring gemessenen Referenzblutglukosemesswerte (BG) des Individuums als Eingangsdaten an das CTP-Eingangsgrößenmodul (E) gelegt werden, dass im CTP-Eingangsgrößenmodul (E) diese Eingangsgrößen entsprechend ihren zugeordneten Sektoren der Selbstkontrolldatentabelle einer Modellparameterkomponente (E1) für die Daten Parameterwerte $b_i$, $a_i$ und k, einer Insulinapplikationskomponente (E2) für die Daten IE und $F_n$, die Nahrungsaufnahmekomponente (E3) für die Daten BE, eine Komponente Applikation oraler Antidiabetika (E4) für die Daten OAD, eine körperliche Aktivitätskomponente (E5) für die Daten IDE und eine Referenzblutglukosemesswertekomponente (E6) für die Daten BG zugeführt und dort entsprechend ihrer Zuordnung als Datensatz im entsprechenden Sektor der Selbstkontrolldatentabelle gespeichert werden, dass ausgangsseitig entsprechend der Zuordnung an der ersten Komponente (E1) eine 24-Stunden-Modellgleichungs-Berechnungseinheit (B1), an der zweiten Komponente (E2) eine 24-Stunden-Insulinwirkprofil-Berechnungseinheit (B2), an der dritten Komponente (E3) eine 24-Stunden-BE-Anflutungs-Berechnungseinheit (B3), an der vierten Komponente (E4) eine 24-Stunden-OAD-Wirkprofil-Berechnungseinheit (B4), an der fünften Komponente (E5) eine 24-Stunden-IDE-Wirkprofil-Berechnungseinheit (B5) und an der sechsten Komponente (E6) eine Referenzblutglukosetagesprofil-Berechnungseinheit (B6) angeschlossen sind, dass der Ausgang der sechs Berechnungseinheiten (B1) bis (B6) an die Eingänge der nachgeordneten CTP-Summations-Berechnungseinheit (B7) im CTP-Berechnungsmodul (B) gekoppelt sind, dass im Ergebnis der Berechnungen im CTP-Berechnungsmodul das persönliche charakteristischen Tagesprofil des individuellen Glukosemetabolismus des Individuums mit seinem 24-Stunden-Blutglukosekonzentrationsverlauf x(t) in Relation zu seinem 24-Stunden-Insulinwirkprofil IE(t), seinem 24-Stunden-Nahrungsanflutiungsprofil BE(t), seinem 24-Stunden-OAD-Wirkprofil OAD(t), seinem 24-Stunden-IDE-Wirkprofil IDE(t) und seinem gemessenen Referenzblutglukosetagesprofil BG(t) vorliegt und über den Ausgang der CTP-Summations-Berechnungseinheit (B7) einer CTP-Darstellungseinheit (A1) für den Metabolischen Fingerabdruck im CTP-Darstellungsmodul (A), die an die CTP-Summations-Berechnungseinheit (B7) angeschlossen ist, übergeben wird.

Fig. 1

Fig. 2

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DD 230730 A

- EP 0834825 B1

**In der Beschreibung aufgeführte Nicht-Patentliteratur**

- Computer Methods and Programs in Biomedicine. Elsevier Science Publishers Biomedical Division, 1990, vol. 32, 215-224